# EUROPEAN PATENT APPLICATION

(11) **EP 2 886 656 A1**
(43) Date of publication of application: **24.06.2015**
(21) Application number: 13306752.0
(22) Date of filing: 18.12.2013
(51) Int. Cl.: C12P 7/22, C12R 1/145, C12N 15/63

(54) **New enzyme and method for preparing 4-hydroxyl benzyl alcohol and derivatives thereof**

(71) Applicant: COMMISSARIAT A L'ENERGIE ATOMIQUE ET AUX ENERGIES ALTERNATIVES, 75015 Paris (FR); Institut National De La Recherche Agronomique, 75007 Paris (FR)
(72) Inventor: Berteau, Olivier, 78350 Jouy en Josas (FR); Nicolet, Yvain, 38220 Vizille (FR); Fontecilla-Camps, Juan, 38190 Les Adrets (FR)
(74) Representative: Gallois, Valérie

(57) **Abstract**

The present invention relates to a new enzyme able to produce 4-hydroxybenzyl alcohol from the amino acid tyrosine and the use thereof for producing 4-hydroxybenzyl alcohol.

SEQUENCE LISTING

<110>  COMMISSARIAT A L'ENERGIE ATOMIQUE ET AUX ENERGIES
       ALTERNATIVES et al

<120>  NEW ENZYME AND METHOD FOR PREPARING 4-HYDROXYL BENZYL ALCOHOL AND
        DERIVATIVES THEREOF

<130>  B1732EP

<160>  12

<170>  PatentIn version 3.3

<210>  1
<211>  1131
<212>  DNA
<213>  Moorella thermoacetica

<220>
<221>  CDS
<222>  (1)..(1131)

<400>  1
atg ggc ttc tat gat gtg tat aaa cag tat gaa ggc ttt gac ttt gaa       48
Met Gly Phe Tyr Asp Val Tyr Lys Gln Tyr Glu Gly Phe Asp Phe Glu
1               5                   10                  15

ggc ttt ttc caa tcg cgc acc ccg gat gac gtt cgt aaa gcg ctg gcc       96
Gly Phe Phe Gln Ser Arg Thr Pro Asp Asp Val Arg Lys Ala Leu Ala
            20                  25                  30

aaa gaa cat ctg gaa gtg acc gat tat ctg acc ctg ctg tct ccg gcc      144
Lys Glu His Leu Glu Val Thr Asp Tyr Leu Thr Leu Leu Ser Pro Ala
        35                  40                  45

gcg ggt aac ttt ctg gaa gaa atg gcc caa aaa gca cac cgt att acc      192
Ala Gly Asn Phe Leu Glu Glu Met Ala Gln Lys Ala His Arg Ile Thr
    50                  55                  60

ctg cgc aat ttc ggt cgt gtc atc ttt ctg ttc acg ccg ctg tat ctg      240
Leu Arg Asn Phe Gly Arg Val Ile Phe Leu Phe Thr Pro Leu Tyr Leu
65                  70                  75                  80

tca gat tac tgc gtg aac cag tgc gcg tat tgt tcg ttt aac gct cgc      288
Ser Asp Tyr Cys Val Asn Gln Cys Ala Tyr Cys Ser Phe Asn Ala Arg
                85                  90                  95

aat aaa ttc gcg cgt acc aaa ctg acg ctg gaa caa gtt gaa gaa gaa      336
Asn Lys Phe Ala Arg Thr Lys Leu Thr Leu Glu Gln Val Glu Glu Glu
            100                 105                 110

gcg cgc gct att gcg cag acc ggc atg aaa gat att ctg atc ctg acg      384
Ala Arg Ala Ile Ala Gln Thr Gly Met Lys Asp Ile Leu Ile Leu Thr
        115                 120                 125

ggt gaa agt cgt caa cat aat ccg gtt tcc tat atc aaa gac tgt gtg      432
Gly Glu Ser Arg Gln His Asn Pro Val Ser Tyr Ile Lys Asp Cys Val
    130                 135                 140

ggc gtt ctg aaa aaa tac ttc tgc agt att tgt atc gaa gtc tat ccg      480
Gly Val Leu Lys Lys Tyr Phe Cys Ser Ile Cys Ile Glu Val Tyr Pro

145                 150                 155                 160

ctg gaa gaa gaa gaa tac cgc gaa ctg gtc gca gct ggc gtg gat ggt       528
Leu Glu Glu Glu Glu Tyr Arg Glu Leu Val Ala Ala Gly Val Asp Gly
                165                 170                 175

ctg acc atg ttc cag gaa gtt tat gac ccg ggc gtc tat gcc cgc tac       576
Leu Thr Met Phe Gln Glu Val Tyr Asp Pro Gly Val Tyr Ala Arg Tyr
            180                 185                 190

cat aac ggt ccg aag aaa aac tat cac tac cgt ctg gat gcg ccg gaa       624
His Asn Gly Pro Lys Lys Asn Tyr His Tyr Arg Leu Asp Ala Pro Glu
        195                 200                 205

cgt agc tgt cgt gcc ggt atg cgt acc gtt ggt gtt ggt gca ctg ctg       672
Arg Ser Cys Arg Ala Gly Met Arg Thr Val Gly Val Gly Ala Leu Leu
    210                 215                 220

ggt ctg gca gac tgg cgt aaa gaa gcg ttt ttc acc ggc ctg cat gca       720
Gly Leu Ala Asp Trp Arg Lys Glu Ala Phe Phe Thr Gly Leu His Ala
225                 230                 235                 240

gat tat ctg cag caa aaa ttt tgg gac gtt caa gtc agt att tcc ctg       768
Asp Tyr Leu Gln Gln Lys Phe Trp Asp Val Gln Val Ser Ile Ser Leu
                245                 250                 255

ccg cgt ttt cgt ccg agc atc ggc ggt ttc caa ccg gat tac ccg gtg       816
Pro Arg Phe Arg Pro Ser Ile Gly Gly Phe Gln Pro Asp Tyr Pro Val
            260                 265                 270

gat gac aaa tct ttt gtt cag att ctg ctg gcc cac cgt ctg ttc ctg       864
Asp Asp Lys Ser Phe Val Gln Ile Leu Leu Ala His Arg Leu Phe Leu
        275                 280                 285

ccg cgt gtg ggt att acc atc tca acg cgc gaa tcg ccg gaa ttt cgt       912
Pro Arg Val Gly Ile Thr Ile Ser Thr Arg Glu Ser Pro Glu Phe Arg
    290                 295                 300

gat aac att ctg ccg ctg ggc gtt acc aaa atc agc gcc ggt agc tct       960
Asp Asn Ile Leu Pro Leu Gly Val Thr Lys Ile Ser Ala Gly Ser Ser
305                 310                 315                 320

gtg acg gtt ggc ggt tat gcg cgt ccg gat ggt atg gcc ccg cag ttc      1008
Val Thr Val Gly Gly Tyr Ala Arg Pro Asp Gly Met Ala Pro Gln Phe
                325                 330                 335

gaa att agc gac ccg cgt tct gtg gcg gaa att aaa cag atg ctg atc      1056
Glu Ile Ser Asp Pro Arg Ser Val Ala Glu Ile Lys Gln Met Leu Ile
            340                 345                 350

caa aaa ggt tac cag ccg gtg ttt gaa gac tgg cag caa tgg gac tca      1104
Gln Lys Gly Tyr Gln Pro Val Phe Glu Asp Trp Gln Gln Trp Asp Ser
        355                 360                 365

ctg gaa aaa caa ctg tat aac ttc taa                                  1131
Leu Glu Lys Gln Leu Tyr Asn Phe
    370                 375

<210>  2
<211>  376
<212>  PRT
<213>  Moorella thermoacetica

<400>  2

Met Gly Phe Tyr Asp Val Tyr Lys Gln Tyr Glu Gly Phe Asp Phe Glu
1               5                   10                  15

Gly Phe Phe Gln Ser Arg Thr Pro Asp Asp Val Arg Lys Ala Leu Ala
            20                  25                  30

Lys Glu His Leu Glu Val Thr Asp Tyr Leu Thr Leu Leu Ser Pro Ala
        35                  40                  45

Ala Gly Asn Phe Leu Glu Glu Met Ala Gln Lys Ala His Arg Ile Thr
    50                  55                  60

Leu Arg Asn Phe Gly Arg Val Ile Phe Leu Phe Thr Pro Leu Tyr Leu
65                  70                  75                  80

Ser Asp Tyr Cys Val Asn Gln Cys Ala Tyr Cys Ser Phe Asn Ala Arg
                85                  90                  95

Asn Lys Phe Ala Arg Thr Lys Leu Thr Leu Glu Gln Val Glu Glu Glu
            100                 105                 110

Ala Arg Ala Ile Ala Gln Thr Gly Met Lys Asp Ile Leu Ile Leu Thr
        115                 120                 125

Gly Glu Ser Arg Gln His Asn Pro Val Ser Tyr Ile Lys Asp Cys Val
    130                 135                 140

Gly Val Leu Lys Lys Tyr Phe Cys Ser Ile Cys Ile Glu Val Tyr Pro
145                 150                 155                 160

Leu Glu Glu Glu Glu Tyr Arg Glu Leu Val Ala Ala Gly Val Asp Gly
                165                 170                 175

Leu Thr Met Phe Gln Glu Val Tyr Asp Pro Gly Val Tyr Ala Arg Tyr
            180                 185                 190

His Asn Gly Pro Lys Lys Asn Tyr His Tyr Arg Leu Asp Ala Pro Glu
        195                 200                 205

Arg Ser Cys Arg Ala Gly Met Arg Thr Val Gly Val Gly Ala Leu Leu
    210                 215                 220

Gly Leu Ala Asp Trp Arg Lys Glu Ala Phe Phe Thr Gly Leu His Ala
225                 230                 235                 240

Asp Tyr Leu Gln Gln Lys Phe Trp Asp Val Gln Val Ser Ile Ser Leu
                245                 250                 255

Pro Arg Phe Arg Pro Ser Ile Gly Gly Phe Gln Pro Asp Tyr Pro Val
            260                 265                 270

Asp Asp Lys Ser Phe Val Gln Ile Leu Leu Ala His Arg Leu Phe Leu
        275                 280                 285

Pro Arg Val Gly Ile Thr Ile Ser Thr Arg Glu Ser Pro Glu Phe Arg
    290                 295                 300

Asp Asn Ile Leu Pro Leu Gly Val Thr Lys Ile Ser Ala Gly Ser Ser
305                 310                 315                 320

Val Thr Val Gly Gly Tyr Ala Arg Pro Asp Gly Met Ala Pro Gln Phe
                325                 330                 335

Glu Ile Ser Asp Pro Arg Ser Val Ala Glu Ile Lys Gln Met Leu Ile
            340                 345                 350

Gln Lys Gly Tyr Gln Pro Val Phe Glu Asp Trp Gln Gln Trp Asp Ser
        355                 360                 365

Leu Glu Lys Gln Leu Tyr Asn Phe
    370                 375

<210>  3
<211>  1068
<212>  DNA
<213>  Chlorobium tepidum

<220>
<221>  CDS
<222>  (1)..(1068)

<400>  3
atg att gcg ctg ccc gca tgg ctg acc gac gag cgg ctg tcg gaa gat        48
Met Ile Ala Leu Pro Ala Trp Leu Thr Asp Glu Arg Leu Ser Glu Asp
1               5                   10                  15

atc gaa ccg ctg ttg cga caa acg gat aac gag tcg ctc gaa cgg ctt        96
Ile Glu Pro Leu Leu Arg Gln Thr Asp Asn Glu Ser Leu Glu Arg Leu
            20                  25                  30

gcc gcc gaa gcg cag gca gtg aca ctg cgc cgt ttc ggg cgc gtc att       144
Ala Ala Glu Ala Gln Ala Val Thr Leu Arg Arg Phe Gly Arg Val Ile
        35                  40                  45

tcg ctc tat acg ccg ctc tac ctc tcc aac ttc tgc tcg agc ggt tgc       192
Ser Leu Tyr Thr Pro Leu Tyr Leu Ser Asn Phe Cys Ser Ser Gly Cys
    50                  55                  60

gtc tat tgc ggc ttc gct tcg gac aga cgt tcg ccg cgc cgc aag ctg 240
Val Tyr Cys Gly Phe Ala Ser Asp Arg Arg Ser Pro Arg Arg Lys Leu
65                  70                  75                  80

gat act gac gaa atc gaa aag gag ctg ctc gca atg aag gct ctc ggc 288
Asp Thr Asp Glu Ile Glu Lys Glu Leu Leu Ala Met Lys Ala Leu Gly
                85                  90                  95

gtc agc gac gtt ttg ctg ctc acc ggc gag cgc acc aac tcg gtg gga 336
Val Ser Asp Val Leu Leu Leu Thr Gly Glu Arg Thr Asn Ser Val Gly
            100                 105                 110

ttc gac tat ctg cgt cgc gcc gtg gat atc gcc gcc cgc cac atg ccg 384
Phe Asp Tyr Leu Arg Arg Ala Val Asp Ile Ala Ala Arg His Met Pro
        115                 120                 125

cgc gta gcc gtc gag gcg ttt ccg atg agc gtc gca gag tat cgc ggc 432
Arg Val Ala Val Glu Ala Phe Pro Met Ser Val Ala Glu Tyr Arg Gly
    130                 135                 140

ctg gcc gaa tgt ggg tgc acc ggc ctg acg att tac cag gaa acc tac 480
Leu Ala Glu Cys Gly Cys Thr Gly Leu Thr Ile Tyr Gln Glu Thr Tyr
145                 150                 155                 160

gat ccg gat cat tac cgc gag ctg cac cgc tgg ggg ccg aag cag gat 528
Asp Pro Asp His Tyr Arg Glu Leu His Arg Trp Gly Pro Lys Gln Asp
                165                 170                 175

ttc ctc gaa cgg ctc gaa acg ccg gaa cgc gcc atc acc ggc ggc atc 576
Phe Leu Glu Arg Leu Glu Thr Pro Glu Arg Ala Ile Thr Gly Gly Ile
            180                 185                 190

cgg agc gtc ggc atc ggc gca ctg ctc ggc ctg tcg gag ccg gtc ggc 624
Arg Ser Val Gly Ile Gly Ala Leu Leu Gly Leu Ser Glu Pro Val Gly
        195                 200                 205

gaa gcg ctc gcc gtg ttg cgc cac gcg cgg tat ctg tgc aaa acg tac 672
Glu Ala Leu Ala Val Leu Arg His Ala Arg Tyr Leu Cys Lys Thr Tyr
    210                 215                 220

tgg aaa gca ggc gtc acg gtc tcc ttt ccc cgc atc cgc ccg cag gag 720
Trp Lys Ala Gly Val Thr Val Ser Phe Pro Arg Ile Arg Pro Gln Glu
225                 230                 235                 240

ggc ggc ttt cag ccc agc ttc acg gtc tcg gat cgc ttc ctc gca cga 768
Gly Gly Phe Gln Pro Ser Phe Thr Val Ser Asp Arg Phe Leu Ala Arg
                245                 250                 255

atg atc ttc gcc ttc cgc atc gga atg ccg gat gtc gat ctg gtg ctc 816
Met Ile Phe Ala Phe Arg Ile Gly Met Pro Asp Val Asp Leu Val Leu
            260                 265                 270

tcg acg cga gag agt tcg aat ttt cgg gac ggc atg gct ggc ctc ggc 864
Ser Thr Arg Glu Ser Ser Asn Phe Arg Asp Gly Met Ala Gly Leu Gly
        275                 280                 285

atc acc cgc atg agc atc gcc agc cgc acc acc gtt ggc ggc tac gtc 912
Ile Thr Arg Met Ser Ile Ala Ser Arg Thr Thr Val Gly Gly Tyr Val
    290                 295                 300

gaa aag gag acg gct gga gcc agc cag ttc gag gtg agc gac aac cga 960
Glu Lys Glu Thr Ala Gly Ala Ser Gln Phe Glu Val Ser Asp Asn Arg
305                 310                 315                 320

agc gtc gaa gcg ttt tgc gcc gca ttg cgc gca aaa gat ctg gaa cca      1008
Ser Val Glu Ala Phe Cys Ala Ala Leu Arg Ala Lys Asp Leu Glu Pro
                325                 330                 335

gtg ttc aaa aac tgg gac gcg gcc tac aac aac ccc ctg ccc gca gag      1056
Val Phe Lys Asn Trp Asp Ala Ala Tyr Asn Asn Pro Leu Pro Ala Glu
            340                 345                 350

gag tgc acg tga                                                      1068
Glu Cys Thr
        355

<210>  4
<211>  355
<212>  PRT
<213>  Chlorobium tepidum

<400>  4

Met Ile Ala Leu Pro Ala Trp Leu Thr Asp Glu Arg Leu Ser Glu Asp
1               5                   10                  15

Ile Glu Pro Leu Leu Arg Gln Thr Asp Asn Glu Ser Leu Glu Arg Leu
            20                  25                  30

Ala Ala Glu Ala Gln Ala Val Thr Leu Arg Arg Phe Gly Arg Val Ile
        35                  40                  45

Ser Leu Tyr Thr Pro Leu Tyr Leu Ser Asn Phe Cys Ser Ser Gly Cys
    50                  55                  60

Val Tyr Cys Gly Phe Ala Ser Asp Arg Arg Ser Pro Arg Arg Lys Leu
65                  70                  75                  80

Asp Thr Asp Glu Ile Glu Lys Glu Leu Leu Ala Met Lys Ala Leu Gly
                85                  90                  95

Val Ser Asp Val Leu Leu Leu Thr Gly Glu Arg Thr Asn Ser Val Gly
            100                 105                 110

Phe Asp Tyr Leu Arg Arg Ala Val Asp Ile Ala Ala Arg His Met Pro
        115                 120                 125

Arg Val Ala Val Glu Ala Phe Pro Met Ser Val Ala Glu Tyr Arg Gly
    130                 135                 140

Leu Ala Glu Cys Gly Cys Thr Gly Leu Thr Ile Tyr Gln Glu Thr Tyr
145                 150                 155                 160

Asp Pro Asp His Tyr Arg Glu Leu His Arg Trp Gly Pro Lys Gln Asp
                165                 170                 175

Phe Leu Glu Arg Leu Glu Thr Pro Glu Arg Ala Ile Thr Gly Gly Ile
            180                 185                 190

Arg Ser Val Gly Ile Gly Ala Leu Leu Gly Leu Ser Glu Pro Val Gly
        195                 200                 205

Glu Ala Leu Ala Val Leu Arg His Ala Arg Tyr Leu Cys Lys Thr Tyr
    210                 215                 220

Trp Lys Ala Gly Val Thr Val Ser Phe Pro Arg Ile Arg Pro Gln Glu
225                 230                 235                 240

Gly Gly Phe Gln Pro Ser Phe Thr Val Ser Asp Arg Phe Leu Ala Arg
                245                 250                 255

Met Ile Phe Ala Phe Arg Ile Gly Met Pro Asp Val Asp Leu Val Leu
            260                 265                 270

Ser Thr Arg Glu Ser Ser Asn Phe Arg Asp Gly Met Ala Gly Leu Gly
        275                 280                 285

Ile Thr Arg Met Ser Ile Ala Ser Arg Thr Thr Val Gly Gly Tyr Val
    290                 295                 300

Glu Lys Glu Thr Ala Gly Ala Ser Gln Phe Glu Val Ser Asp Asn Arg
305                 310                 315                 320

Ser Val Glu Ala Phe Cys Ala Ala Leu Arg Ala Lys Asp Leu Glu Pro
                325                 330                 335

Val Phe Lys Asn Trp Asp Ala Ala Tyr Asn Asn Pro Leu Pro Ala Glu
            340                 345                 350

Glu Cys Thr
        355

<210>  5
<211>  1122
<212>  DNA
<213>  Carboxythermus hydrogenoformans

<220>
<221>  CDS
<222>  (1)..(1122)

<400>  5
atg ctg gac tac ctg caa aaa tgc ttt gaa ctg tac ttt cgt tat gaa        48
Met Leu Asp Tyr Leu Gln Lys Cys Phe Glu Leu Tyr Phe Arg Tyr Glu

1               5                   10                  15

caa tac gtg ccg tcg ttt agc gaa gct ctg gac atc ctg gcg aaa gat      96
Gln Tyr Val Pro Ser Phe Ser Glu Ala Leu Asp Ile Leu Ala Lys Asp
            20                  25                  30

tat ctg gac aaa att gat ctg gtt aaa ctg ctg aac gtc gaa gat gaa      144
Tyr Leu Asp Lys Ile Asp Leu Val Lys Leu Leu Asn Val Glu Asp Glu
        35                  40                  45

gaa atc atc aaa ttc atg gcg aaa aaa gcc aaa cgt atc acc gaa ctg      192
Glu Ile Ile Lys Phe Met Ala Lys Lys Ala Lys Arg Ile Thr Glu Leu
    50                  55                  60

aat ttc ggc aaa gtg att ctg ctg tat gca ccg ctg tac atc gct aac      240
Asn Phe Gly Lys Val Ile Leu Leu Tyr Ala Pro Leu Tyr Ile Ala Asn
65                  70                  75                  80

ttt tgc gaa aat ggc tgc gtt tat tgt ggt ttc tct aaa ctg cgt aaa      288
Phe Cys Glu Asn Gly Cys Val Tyr Cys Gly Phe Ser Lys Leu Arg Lys
                85                  90                  95

tac ccg cgc gaa aaa ctg agt ctg gaa cag atg gaa gaa gaa atg cag      336
Tyr Pro Arg Glu Lys Leu Ser Leu Glu Gln Met Glu Glu Glu Met Gln
            100                 105                 110

caa att aaa tca gaa ggc att gac tcg atc ctg ctg ctg acc ggt gaa      384
Gln Ile Lys Ser Glu Gly Ile Asp Ser Ile Leu Leu Leu Thr Gly Glu
        115                 120                 125

gat cgt aaa aac agc ccg ttc gca tac atc aaa aac gca tgt aaa ctg      432
Asp Arg Lys Asn Ser Pro Phe Ala Tyr Ile Lys Asn Ala Cys Lys Leu
    130                 135                 140

gct acg aaa tac ttc agc gaa gtc tct atc gaa gtg tat ccg ctg agt      480
Ala Thr Lys Tyr Phe Ser Glu Val Ser Ile Glu Val Tyr Pro Leu Ser
145                 150                 155                 160

aaa gaa gaa tac gaa gaa ctg gct cgc att ggc gtt atc ggt acc acg      528
Lys Glu Glu Tyr Glu Glu Leu Ala Arg Ile Gly Val Ile Gly Thr Thr
                165                 170                 175

att tat cag gaa acc tac atc aaa aaa gac tac gaa aaa ctg cac ctg      576
Ile Tyr Gln Glu Thr Tyr Ile Lys Lys Asp Tyr Glu Lys Leu His Leu
            180                 185                 190

ttt ggc ccg aaa aaa gat tat gaa ttt cgt ctg tac acg ccg gaa cgc      624
Phe Gly Pro Lys Lys Asp Tyr Glu Phe Arg Leu Tyr Thr Pro Glu Arg
        195                 200                 205

gca ctg aaa gct ggt ttt aaa gcg gcc tca att ggc ccg ctg ctg ggt      672
Ala Leu Lys Ala Gly Phe Lys Ala Ala Ser Ile Gly Pro Leu Leu Gly
    210                 215                 220

ctg tcg ctg ccg aaa ctg gac gtg tat agc gcg atc ctg cat gcc gat      720
Leu Ser Leu Pro Lys Leu Asp Val Tyr Ser Ala Ile Leu His Ala Asp
225                 230                 235                 240

tat ctg atg aaa aaa tac ccg caa gcg gaa att gcc atc tcc ttt ccg      768
Tyr Leu Met Lys Lys Tyr Pro Gln Ala Glu Ile Ala Ile Ser Phe Pro
                245                 250                 255

cgt ctg cgc gca gct aac acc ggc ttc aaa gca aaa cac gtg gtt tct      816

Arg Leu Arg Ala Ala Asn Thr Gly Phe Lys Ala Lys His Val Val Ser
            260                 265                 270

gat aaa gaa ttt atc aaa ttc ctg ctg gtc acc cgt atc tat ctg ccg      864
Asp Lys Glu Phe Ile Lys Phe Leu Leu Val Thr Arg Ile Tyr Leu Pro
        275                 280                 285

cgc att ggt atc aat ctg agt acc cgc gaa cgc ccg tcc ctg cgt gat      912
Arg Ile Gly Ile Asn Leu Ser Thr Arg Glu Arg Pro Ser Leu Arg Asp
    290                 295                 300

gcc ctg ctg gat att tgc atc acc aaa atg agt gcc ggc tcc aaa acc      960
Ala Leu Leu Asp Ile Cys Ile Thr Lys Met Ser Ala Gly Ser Lys Thr
305                 310                 315                 320

acg gtg ggc ggt tac ttt agc aaa aaa gaa gac tct cag ggt caa ttc      1008
Thr Val Gly Gly Tyr Phe Ser Lys Lys Glu Asp Ser Gln Gly Gln Phe
                325                 330                 335

gaa gtg gaa gat cgt cgc atg gtt gcc gaa att atc gaa gtc att cgc      1056
Glu Val Glu Asp Arg Arg Met Val Ala Glu Ile Ile Glu Val Ile Arg
            340                 345                 350

aaa aaa ggt ctg cgc ccg gaa ttt acc aac tgg att cgc ggt gtt cgc      1104
Lys Lys Gly Leu Arg Pro Glu Phe Thr Asn Trp Ile Arg Gly Val Arg
        355                 360                 365

ccg tat gaa ctg ctg tga                                              1122
Pro Tyr Glu Leu Leu
    370

<210>  6
<211>  373
<212>  PRT
<213>  Carboxythermus hydrogenoformans

<400>  6

Met Leu Asp Tyr Leu Gln Lys Cys Phe Glu Leu Tyr Phe Arg Tyr Glu
1               5                   10                  15

Gln Tyr Val Pro Ser Phe Ser Glu Ala Leu Asp Ile Leu Ala Lys Asp
            20                  25                  30

Tyr Leu Asp Lys Ile Asp Leu Val Lys Leu Leu Asn Val Glu Asp Glu
        35                  40                  45

Glu Ile Ile Lys Phe Met Ala Lys Lys Ala Lys Arg Ile Thr Glu Leu
    50                  55                  60

Asn Phe Gly Lys Val Ile Leu Leu Tyr Ala Pro Leu Tyr Ile Ala Asn
65                  70                  75                  80

Phe Cys Glu Asn Gly Cys Val Tyr Cys Gly Phe Ser Lys Leu Arg Lys
                85                  90                  95

Tyr Pro Arg Glu Lys Leu Ser Leu Glu Gln Met Glu Glu Glu Met Gln
        100                 105                 110

Gln Ile Lys Ser Glu Gly Ile Asp Ser Ile Leu Leu Leu Thr Gly Glu
        115                 120                 125

Asp Arg Lys Asn Ser Pro Phe Ala Tyr Ile Lys Asn Ala Cys Lys Leu
    130                 135                 140

Ala Thr Lys Tyr Phe Ser Glu Val Ser Ile Glu Val Tyr Pro Leu Ser
145                 150                 155                 160

Lys Glu Glu Tyr Glu Glu Leu Ala Arg Ile Gly Val Ile Gly Thr Thr
                165                 170                 175

Ile Tyr Gln Glu Thr Tyr Ile Lys Lys Asp Tyr Glu Lys Leu His Leu
            180                 185                 190

Phe Gly Pro Lys Lys Asp Tyr Glu Phe Arg Leu Tyr Thr Pro Glu Arg
        195                 200                 205

Ala Leu Lys Ala Gly Phe Lys Ala Ala Ser Ile Gly Pro Leu Leu Gly
    210                 215                 220

Leu Ser Leu Pro Lys Leu Asp Val Tyr Ser Ala Ile Leu His Ala Asp
225                 230                 235                 240

Tyr Leu Met Lys Lys Tyr Pro Gln Ala Glu Ile Ala Ile Ser Phe Pro
                245                 250                 255

Arg Leu Arg Ala Ala Asn Thr Gly Phe Lys Ala Lys His Val Val Ser
            260                 265                 270

Asp Lys Glu Phe Ile Lys Phe Leu Leu Val Thr Arg Ile Tyr Leu Pro
        275                 280                 285

Arg Ile Gly Ile Asn Leu Ser Thr Arg Glu Arg Pro Ser Leu Arg Asp
    290                 295                 300

Ala Leu Leu Asp Ile Cys Ile Thr Lys Met Ser Ala Gly Ser Lys Thr
305                 310                 315                 320

Thr Val Gly Gly Tyr Phe Ser Lys Lys Glu Asp Ser Gln Gly Gln Phe
                325                 330                 335

Glu Val Glu Asp Arg Arg Met Val Ala Glu Ile Ile Glu Val Ile Arg
            340                 345                 350

Lys Lys Gly Leu Arg Pro Glu Phe Thr Asn Trp Ile Arg Gly Val Arg
        355                 360                 365

Pro Tyr Glu Leu Leu
    370

<210>  7
<211>  1107
<212>  DNA
<213>  Clostridium acetobutylicum

<220>
<221>  CDS
<222>  (1)..(1107)

<400>  7
atg agc ttc tac aaa aaa ctg caa caa tac aaa gac ttc gat ttc gac      48
Met Ser Phe Tyr Lys Lys Leu Gln Gln Tyr Lys Asp Phe Asp Phe Asp
1               5                   10                  15

gac ttc ttc agc aaa gtg acg gca cgc gac att gaa aaa atc ctg tgc      96
Asp Phe Phe Ser Lys Val Thr Ala Arg Asp Ile Glu Lys Ile Leu Cys
            20                  25                  30

aaa gat att ctg cat gaa atg gac ttt ctg aaa ctg ctg agc ccg gcg      144
Lys Asp Ile Leu His Glu Met Asp Phe Leu Lys Leu Leu Ser Pro Ala
        35                  40                  45

gcc gaa aaa tac ctg gaa cac atg gca cag aaa gct cgt gaa ctg tct      192
Ala Glu Lys Tyr Leu Glu His Met Ala Gln Lys Ala Arg Glu Leu Ser
    50                  55                  60

ctg aaa aac ttc ggc aaa acc gtg gtt ctg tat acg ccg att tac atc      240
Leu Lys Asn Phe Gly Lys Thr Val Val Leu Tyr Thr Pro Ile Tyr Ile
65                  70                  75                  80

gca aac tat tgt gtg aat ggc tgc gct tac tgt ggt tac aac gtt aaa      288
Ala Asn Tyr Cys Val Asn Gly Cys Ala Tyr Cys Gly Tyr Asn Val Lys
                85                  90                  95

aac aaa att aaa cgc aaa cag ctg acg atg gaa gaa atc gaa gaa gaa      336
Asn Lys Ile Lys Arg Lys Gln Leu Thr Met Glu Glu Ile Glu Glu Glu
            100                 105                 110

gcg cgt gct att tat agc tct ggc atg cgc aac att atc ctg ctg acc      384
Ala Arg Ala Ile Tyr Ser Ser Gly Met Arg Asn Ile Ile Leu Leu Thr
        115                 120                 125

ggt gaa agc aaa gtg caa acg ccg gtt tct tac atc aaa gat gcg atc      432
Gly Glu Ser Lys Val Gln Thr Pro Val Ser Tyr Ile Lys Asp Ala Ile
    130                 135                 140

aaa ctg ctg aaa aaa tac ttc agt tcc att tgc atc gaa att tac ccg      480
Lys Leu Leu Lys Lys Tyr Phe Ser Ser Ile Cys Ile Glu Ile Tyr Pro
145                 150                 155                 160

ctg gaa gtt aat gaa tat cgt gaa ctg gtc gaa gcg ggc gcc gat agc      528
Leu Glu Val Asn Glu Tyr Arg Glu Leu Val Glu Ala Gly Ala Asp Ser
                165                 170                 175

ctg acc atc tac caa gaa acg tac aac gaa gaa aaa tac tca aaa gtc 576
Leu Thr Ile Tyr Gln Glu Thr Tyr Asn Glu Glu Lys Tyr Ser Lys Val
            180                 185                 190

cac ctg tcg ggt ccg aaa cgt aat ttt aaa ttc cgc ctg gac gcg ccg 624
His Leu Ser Gly Pro Lys Arg Asn Phe Lys Phe Arg Leu Asp Ala Pro
        195                 200                 205

gaa cgt gtt tgt gaa gcc ggc atc cat tca att ggc acc ggt gcc ctg 672
Glu Arg Val Cys Glu Ala Gly Ile His Ser Ile Gly Thr Gly Ala Leu
    210                 215                 220

ctg ggt ctg tac aaa tgg cgc tcg gaa gca ttt ttc acg ggc ctg cac 720
Leu Gly Leu Tyr Lys Trp Arg Ser Glu Ala Phe Phe Thr Gly Leu His
225                 230                 235                 240

gct agt tat atc cag gaa aaa ttt ccg tcc gtg gaa atc tca atg agc 768
Ala Ser Tyr Ile Gln Glu Lys Phe Pro Ser Val Glu Ile Ser Met Ser
                245                 250                 255

gcc ccg cgt att cgc ccg cat gca ggt agc ttc gat gac atc tac gaa 816
Ala Pro Arg Ile Arg Pro His Ala Gly Ser Phe Asp Asp Ile Tyr Glu
            260                 265                 270

gtt aac gat aaa aac atc gtc caa gtg att ctg gcc tat aaa atg ttt 864
Val Asn Asp Lys Asn Ile Val Gln Val Ile Leu Ala Tyr Lys Met Phe
        275                 280                 285

ctg ccg cgt gca ggc acc aac atc acc acg cgt gaa ccg aaa gaa ttt 912
Leu Pro Arg Ala Gly Thr Asn Ile Thr Thr Arg Glu Pro Lys Glu Phe
    290                 295                 300

cgc gat aaa ctg atc ccg att ggc gtg acc aaa atg agt gcg ggt gtc 960
Arg Asp Lys Leu Ile Pro Ile Gly Val Thr Lys Met Ser Ala Gly Val
305                 310                 315                 320

tcc acg gaa gtg ggc ggt cat ggt tgc aaa gac aaa ggc gaa ggc cag 1008
Ser Thr Glu Val Gly Gly His Gly Cys Lys Asp Lys Gly Glu Gly Gln
                325                 330                 335

ttc gat acc aat gac aaa cgc agc gtt tct gaa gtc tat aat cgt atc 1056
Phe Asp Thr Asn Asp Lys Arg Ser Val Ser Glu Val Tyr Asn Arg Ile
            340                 345                 350

aaa gaa ctg ggc tac aac ccg gtg ttc aaa gac ttc gaa aac gca ctg 1104
Lys Glu Leu Gly Tyr Asn Pro Val Phe Lys Asp Phe Glu Asn Ala Leu
        355                 360                 365

tga 1107

<210> 8
<211> 368
<212> PRT
<213> Clostridium acetobutylicum

<400> 8

Met Ser Phe Tyr Lys Lys Leu Gln Gln Tyr Lys Asp Phe Asp Phe Asp
1               5                   10                  15

Asp Phe Phe Ser Lys Val Thr Ala Arg Asp Ile Glu Lys Ile Leu Cys
        20                  25                  30

Lys Asp Ile Leu His Glu Met Asp Phe Leu Lys Leu Leu Ser Pro Ala
    35                  40                  45

Ala Glu Lys Tyr Leu Glu His Met Ala Gln Lys Ala Arg Glu Leu Ser
    50                  55                  60

Leu Lys Asn Phe Gly Lys Thr Val Val Leu Tyr Thr Pro Ile Tyr Ile
65                  70                  75                  80

Ala Asn Tyr Cys Val Asn Gly Cys Ala Tyr Cys Gly Tyr Asn Val Lys
                85                  90                  95

Asn Lys Ile Lys Arg Lys Gln Leu Thr Met Glu Glu Ile Glu Glu Glu
            100                 105                 110

Ala Arg Ala Ile Tyr Ser Ser Gly Met Arg Asn Ile Ile Leu Leu Thr
        115                 120                 125

Gly Glu Ser Lys Val Gln Thr Pro Val Ser Tyr Ile Lys Asp Ala Ile
    130                 135                 140

Lys Leu Leu Lys Lys Tyr Phe Ser Ser Ile Cys Ile Glu Ile Tyr Pro
145                 150                 155                 160

Leu Glu Val Asn Glu Tyr Arg Glu Leu Val Glu Ala Gly Ala Asp Ser
                165                 170                 175

Leu Thr Ile Tyr Gln Glu Thr Tyr Asn Glu Glu Lys Tyr Ser Lys Val
            180                 185                 190

His Leu Ser Gly Pro Lys Arg Asn Phe Lys Phe Arg Leu Asp Ala Pro
        195                 200                 205

Glu Arg Val Cys Glu Ala Gly Ile His Ser Ile Gly Thr Gly Ala Leu
    210                 215                 220

Leu Gly Leu Tyr Lys Trp Arg Ser Glu Ala Phe Phe Thr Gly Leu His
225                 230                 235                 240

Ala Ser Tyr Ile Gln Glu Lys Phe Pro Ser Val Glu Ile Ser Met Ser
                245                 250                 255

Ala Pro Arg Ile Arg Pro His Ala Gly Ser Phe Asp Asp Ile Tyr Glu
            260                 265                 270

Val Asn Asp Lys Asn Ile Val Gln Val Ile Leu Ala Tyr Lys Met Phe
        275                 280                 285

Leu Pro Arg Ala Gly Thr Asn Ile Thr Thr Arg Glu Pro Lys Glu Phe
    290                 295                 300

Arg Asp Lys Leu Ile Pro Ile Gly Val Thr Lys Met Ser Ala Gly Val
305                 310                 315                 320

Ser Thr Glu Val Gly Gly His Gly Cys Lys Asp Lys Gly Glu Gly Gln
                325                 330                 335

Phe Asp Thr Asn Asp Lys Arg Ser Val Ser Glu Val Tyr Asn Arg Ile
            340                 345                 350

Lys Glu Leu Gly Tyr Asn Pro Val Phe Lys Asp Phe Glu Asn Ala Leu
        355                 360                 365

<210>  9
<211>  1134
<212>  DNA
<213>  Escherichia coli

<220>
<221>  CDS
<222>  (1)..(1134)

<400>  9
atg aaa acc ttc agc gat cgc tgg cga caa ctg gac tgg gac gac atc      48
Met Lys Thr Phe Ser Asp Arg Trp Arg Gln Leu Asp Trp Asp Asp Ile
1               5                   10                  15

cgc ctg cgt atc aac ggc aaa acg gct gct gac gta gag cgg gcg cta      96
Arg Leu Arg Ile Asn Gly Lys Thr Ala Ala Asp Val Glu Arg Ala Leu
            20                  25                  30

aat gcc tcg caa ctc acc cgc gac gac atg atg gcg ctg tta tcg cct      144
Asn Ala Ser Gln Leu Thr Arg Asp Asp Met Met Ala Leu Leu Ser Pro
        35                  40                  45

gcc gcc agt ggc tat ctg gaa caa ctg gcc caa cgg gcg cag cgt ctg      192
Ala Ala Ser Gly Tyr Leu Glu Gln Leu Ala Gln Arg Ala Gln Arg Leu
    50                  55                  60

acc cgt cag cga ttt ggc aac aca gtt agt ttc tac gtc ccg ctt tat      240
Thr Arg Gln Arg Phe Gly Asn Thr Val Ser Phe Tyr Val Pro Leu Tyr
65                  70                  75                  80

ctt tcc aat ctt tgc gct aac gac tgc acg tac tgt gga ttt tcc atg      288
Leu Ser Asn Leu Cys Ala Asn Asp Cys Thr Tyr Cys Gly Phe Ser Met
                85                  90                  95

agt aat cgc atc aag cgc aaa acg ctg gat gaa gcg gat att gcc agg      336
Ser Asn Arg Ile Lys Arg Lys Thr Leu Asp Glu Ala Asp Ile Ala Arg
            100                 105                 110

gaa agt gcc gct ata cgg gag atg ggc ttt gaa cat ctg ctg tta gtc     384
Glu Ser Ala Ala Ile Arg Glu Met Gly Phe Glu His Leu Leu Leu Val
        115                 120                 125

act ggt gaa cat cag gcg aaa gtg ggg atg gat tac ttt cgt cgt cat     432
Thr Gly Glu His Gln Ala Lys Val Gly Met Asp Tyr Phe Arg Arg His
    130                 135                 140

ctc cct gcc ctt cgt gaa cag ttc tct tca cta cag atg gaa gtg caa     480
Leu Pro Ala Leu Arg Glu Gln Phe Ser Ser Leu Gln Met Glu Val Gln
145                 150                 155                 160

ccg ctg gcg gag acg gaa tac gcc gag tta aag caa ctt ggt ctg gat     528
Pro Leu Ala Glu Thr Glu Tyr Ala Glu Leu Lys Gln Leu Gly Leu Asp
                165                 170                 175

ggc gtg atg gtt tat cag gag aca tat cac gag gcg act tat gcc cgc     576
Gly Val Met Val Tyr Gln Glu Thr Tyr His Glu Ala Thr Tyr Ala Arg
            180                 185                 190

cat cat ctg aaa ggc aaa aaa cag gac ttc ttc tgg cgg ctg gaa acg     624
His His Leu Lys Gly Lys Lys Gln Asp Phe Phe Trp Arg Leu Glu Thr
        195                 200                 205

ccg gat cgg ctg ggg cgt gcg ggg att gat aag ata ggc ctc ggc gcg     672
Pro Asp Arg Leu Gly Arg Ala Gly Ile Asp Lys Ile Gly Leu Gly Ala
    210                 215                 220

cta att ggc ctt tcc gac aac tgg cgc gtt gac agc tat atg gtt gcc     720
Leu Ile Gly Leu Ser Asp Asn Trp Arg Val Asp Ser Tyr Met Val Ala
225                 230                 235                 240

gaa cat ttg cta tgg ctg caa cag cat tac tgg caa agc cgt tac tct     768
Glu His Leu Leu Trp Leu Gln Gln His Tyr Trp Gln Ser Arg Tyr Ser
                245                 250                 255

gtc tcc ttt ccg cgc ctg cgc ccg tgt act ggc ggc att gag cct gcg     816
Val Ser Phe Pro Arg Leu Arg Pro Cys Thr Gly Gly Ile Glu Pro Ala
            260                 265                 270

tcg att atg gat gaa cgc cag tta gtg caa acc atc tgc gcc ttc cga     864
Ser Ile Met Asp Glu Arg Gln Leu Val Gln Thr Ile Cys Ala Phe Arg
        275                 280                 285

ctg ctt gca ccg gag att gaa ctg tca ctc tcc acg cgg gaa tca ccg     912
Leu Leu Ala Pro Glu Ile Glu Leu Ser Leu Ser Thr Arg Glu Ser Pro
    290                 295                 300

tgg ttt cgc gat cgc gtt att ccg ctg gcg atc aat aac gtc agc gcc     960
Trp Phe Arg Asp Arg Val Ile Pro Leu Ala Ile Asn Asn Val Ser Ala
305                 310                 315                 320

ttc tcg aaa acg cag cca ggt ggc tat gcc gat aat cac ccc gag ttg     1008
Phe Ser Lys Thr Gln Pro Gly Gly Tyr Ala Asp Asn His Pro Glu Leu
                325                 330                 335

gaa cag ttc tca ccg cac gac gat cgc aga ccg gaa gcg gtt gct gcc     1056
Glu Gln Phe Ser Pro His Asp Asp Arg Arg Pro Glu Ala Val Ala Ala
            340                 345                 350

gcg tta acc gct cag ggt ttg cag ccg gta tgg aaa gac tgg gac agc     1104
Ala Leu Thr Ala Gln Gly Leu Gln Pro Val Trp Lys Asp Trp Asp Ser

355                 360                 365

tat ctg gga cgc gcc tcg caa aga cta tga                              1134
Tyr Leu Gly Arg Ala Ser Gln Arg Leu
    370                 375

<210>  10
<211>  377
<212>  PRT
<213>  Escherichia coli

<400>  10

Met Lys Thr Phe Ser Asp Arg Trp Arg Gln Leu Asp Trp Asp Asp Ile
1               5                   10                  15

Arg Leu Arg Ile Asn Gly Lys Thr Ala Ala Asp Val Glu Arg Ala Leu
            20                  25                  30

Asn Ala Ser Gln Leu Thr Arg Asp Asp Met Met Ala Leu Leu Ser Pro
        35                  40                  45

Ala Ala Ser Gly Tyr Leu Glu Gln Leu Ala Gln Arg Ala Gln Arg Leu
    50                  55                  60

Thr Arg Gln Arg Phe Gly Asn Thr Val Ser Phe Tyr Val Pro Leu Tyr
65                  70                  75                  80

Leu Ser Asn Leu Cys Ala Asn Asp Cys Thr Tyr Cys Gly Phe Ser Met
                85                  90                  95

Ser Asn Arg Ile Lys Arg Lys Thr Leu Asp Glu Ala Asp Ile Ala Arg
            100                 105                 110

Glu Ser Ala Ala Ile Arg Glu Met Gly Phe Glu His Leu Leu Leu Val
        115                 120                 125

Thr Gly Glu His Gln Ala Lys Val Gly Met Asp Tyr Phe Arg Arg His
    130                 135                 140

Leu Pro Ala Leu Arg Glu Gln Phe Ser Ser Leu Gln Met Glu Val Gln
145                 150                 155                 160

Pro Leu Ala Glu Thr Glu Tyr Ala Glu Leu Lys Gln Leu Gly Leu Asp
                165                 170                 175

Gly Val Met Val Tyr Gln Glu Thr Tyr His Glu Ala Thr Tyr Ala Arg
            180                 185                 190

His His Leu Lys Gly Lys Lys Gln Asp Phe Phe Trp Arg Leu Glu Thr

195                 200                 205

Pro Asp Arg Leu Gly Arg Ala Gly Ile Asp Lys Ile Gly Leu Gly Ala
    210                 215                 220

Leu Ile Gly Leu Ser Asp Asn Trp Arg Val Asp Ser Tyr Met Val Ala
225                 230                 235                 240

Glu His Leu Leu Trp Leu Gln Gln His Tyr Trp Gln Ser Arg Tyr Ser
                245                 250                 255

Val Ser Phe Pro Arg Leu Arg Pro Cys Thr Gly Gly Ile Glu Pro Ala
            260                 265                 270

Ser Ile Met Asp Glu Arg Gln Leu Val Gln Thr Ile Cys Ala Phe Arg
        275                 280                 285

Leu Leu Ala Pro Glu Ile Glu Leu Ser Leu Ser Thr Arg Glu Ser Pro
    290                 295                 300

Trp Phe Arg Asp Arg Val Ile Pro Leu Ala Ile Asn Asn Val Ser Ala
305                 310                 315                 320

Phe Ser Lys Thr Gln Pro Gly Gly Tyr Ala Asp Asn His Pro Glu Leu
                325                 330                 335

Glu Gln Phe Ser Pro His Asp Asp Arg Arg Pro Glu Ala Val Ala Ala
            340                 345                 350

Ala Leu Thr Ala Gln Gly Leu Gln Pro Val Trp Lys Asp Trp Asp Ser
        355                 360                 365

Tyr Leu Gly Arg Ala Ser Gln Arg Leu
    370                 375

<210>  11
<211>  37
<212>  DNA
<213>  artificial sequence

<220>
<223>  primer

<400>  11
ggtaatccat atgattgcgc tgcccgcatg gctgacc                              37

<210>  12
<211>  36
<212>  DNA
<213>  artificial sequence

<220>
<223>  primer

<400>  12
gggaattctt atcacgtgca ctcctctgcg ggcagg                                36

## Description

### FIELD OF THE INVENTION

The present invention relates to a new enzyme and its use in methods for preparing compounds of interest.

### BACKGROUND OF THE INVENTION

Aromatic compounds, including hydroxyl benzyl alcohols (HBA's), which are intermediates in the manufacturing of dyes, pharmaceutical products, additives, and polymers, are key elements in the chemical industry. Furthermore, HBA's have interesting biological functions and properties, exhibiting notably an excellent neuroprotective effect and are effective free radical scavengers.

**p-Hydroxybenzyl alcohol** (4-HBA) and its derivatives are important starting materials for the synthesis of useful organic compounds including pharmaceutical compounds such as the cardioselective β1-adrenergic blocking agent bisoprolol (WO/2007/069266 / Arcelor Ltd); vanillin (Rhodia), various chemicals such as p-hydroxybenzylaldehyde, 4,4'-dihydroxydiphenylmethane and polymers. For instance, 4-HBA can be used to prepare liquid-crystalline polymer (e.g., US2012/190813). Furthermore, 4-HBA has been shown to possess anti-angiogenic, anti-inflammatory and anti-nociceptive activities and, for instance, it has been patented to treat ischemic brain disease (WO2005/030189).

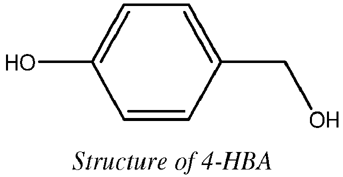

The global market for vanillin, the world's most popular flavor, for which 4-HBA is a precursor, is estimated to be between 15-16,000 tons per year.

The market for 4-HBA is thus extensive covering the production of food, polymers and pharmaceutical compounds. Different quality grades of 4-HBA are required, notably for medical applications. However, the production of 4-HBA remains difficult.

An important goal in synthetic chemistry is to develop environmentally friendly and increasingly safer processes. HBA's are generally synthesized by reduction of the corresponding aromatic aldehydes. 4-HBA is industrially produced by the reaction of phenol with formaldehyde in the presence of a basic catalyst. Through this process, a mixture of 4-HBA (p-hydroxybenzyl alcohol) and o-hydroxybenzyl alcohol is obtained and the two isomers have to be separated. The o-hydroxybenzyl alcohol is predominantly formed and the addition of various solvents is commonly used to increase the amount of 4-HBA produced. The isolation of the pure compounds from these reaction mixtures is further complicated by the formation of by-products. Indeed, not only these two isomers are produced but, as a result of their high reactivity, the hydroxybenzyl alcohols react with the formaldehyde present in the reaction mixture and self-condensation can also occur.

Many improvements have been introduced to this process (US Patent 4,205,188), notably the addition of catalyst (US Patent 5,019,656). However, it still requires the use of large amounts of organic compounds and solvent and involves several purification steps.

These problems notwithstanding, few biotechnological alternatives have been developed to safely produce renewable 4-HBA within the frame of a 'green chemistry' approach.

Bacterial production of 4-HBA has been described recently, involving either an increased production of aromatic amino acids or a reduction of their use by the host cell. A bacterial cell, which has an increased flux in the biosynthesis of one or more aromatic amino acids has been disclosed (EP1764415). Major disadvantages of this approach include the need to purify 4-HBA from the bacteria metabolites and its empirical nature, with no biosynthetic pathway clearly identified.

Alternatively, several biosynthetic pathways have been disclosed that, theoretically, could lead to the production of 4-HBA. However, they have not been disclosed as such and it is not clear whether 4-HBA could be really isolated therefrom.

In plants, the chain shortening of p-coumaric acid to p-hydroxybenzaldehyde has been disclosed in *Vanilla planifolia* (US2003/0070188).

In bacteria, a method for the production of p-hydroxybenzoate in species of *Pseudomonas* and *Agrobacterium* has been disclosed (EP1292682). The p-cresol methylhydroxylase (PCMH) converts p-cresol to 4-HBA and further oxidizes it to p-hydroxybenzoate.

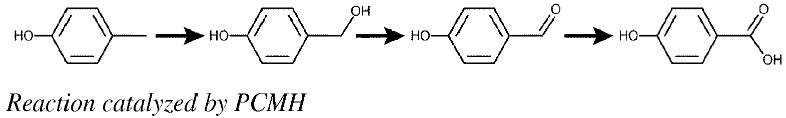

p-Cresol has problems in term of stability and toxicity. But most importantly, PCMH uses 4-HBA as a substrate and further oxidizes it making it unsuitable for 4-HBA production.. Thus, the preparation of 4-HBA by this process would involve its costly regeneration from p-hydroxybenzoate.

In conclusion, there is an urgent need for a new method for producing efficiently 4-HBA, while both limiting the use of organic solvents and facilitating its purification.

### SUMMARY OF THE INVENTION

The present invention relates to the discovery that a ThiH (tyrosine lyase) enzyme from the thermophilic bacterium *Moorella thermoacetica* having the amino acid sequence of SEQ ID No 2 is capable of producing 4-HBA from tyrosine in an efficient manner. Its ability to catalyze this reaction is really surprising because the well-know homologous ThiH enzyme from *E coli* has been reported to be unable to produce 4-HBA (Kriek et al. 2007 Angew Chem Int Ed Engl. 2007;46(48):9223-6). Thus, the use of this enzyme represents a novel way to produce 4-HBA through safer and sustainable production processes involving only one step from tyrosine.

It is thus provided an isolated or recombinant enzyme comprising an amino sequence having at least 80 % identity with SEQ ID No 2 and being capable of producing 4-HBA and p-cresol from L-tyrosine. Preferably, the enzyme comprises or consists of an amino sequence having at least 90, 95, 97.5 or 99 % identity with SEQ ID No 2. More particularly, the enzyme may comprise or consist of the amino sequence of SEQ ID No 2.

It is also provided a composition or a kit comprising the isolated or recombinant enzyme as defined above and a solid support on which is immobilized the enzyme as defined above. In particular, the composition may include iron and sulfur as enzyme additives and a reducing agent such as dithiothreitol or beta-mercaptoethanol. In addition the composition may include S-adenosyl L-methionine (SAM), the enzyme cofactor or methionine and ATP when in the presence of SAM synthase.

It is further provided a recombinant nucleic acid construct or vector comprising a nucleic acid sequence encoding the enzyme as defined above. More particularly, the nucleic acid construct or vector is suitable for expressing the said enzyme. In addition, it is provided a recombinant host cell comprising a nucleic acid, a recombinant nucleic acid construct or a recombinant vector comprising a nucleic acid sequence encoding the enzyme as defined above.

It is provided a method for producing an enzyme capable of making 4-HBA and p-cresol from L-tyrosine, comprising culturing the host cell as defined above, under conditions conducive to the production of the enzyme, and recovering and/or purifying the enzyme. Alternatively, it is also provided a method for producing an enzyme capable of making 4-HBA and p-cresol from L-tyrosine, comprising the *in vitro* expression of the enzyme with a nucleic acid encoding the enzyme as defined above. Optionally, the method further comprises a step of immobilizing the enzyme on a solid support.

The present invention also relates to the use of an enzyme as defined above, a composition, kit or solid support comprising the enzyme, or a recombinant host cell comprising a nucleic acid, a recombinant nucleic acid construct or a recombinant vector comprising a nucleic acid sequence encoding the enzyme as defined above, for producing 4-hydroxyl benzyl alcohol (4-HBA) or an analog thereof.

Accordingly, the present invention relates to a method for producing 4-hydroxyl benzyl alcohol (4-HBA) or an analog thereof comprising contacting tyrosine or an analog thereof with an enzyme comprising an amino sequence having at least 80 % identity with SEQ ID No 2 and being capable of producing 4-HBA and p-cresol from L-tyrosine, and optionally recovering 4-HBA or the analog thereof.

Finally, the present invention relates to a method for producing a compound of interest, comprising producing 4-HBA or an analog thereof by the method according to the present disclosure and using the 4-HBA or the analog thereof for producing the compound of interest. Optionally, the compound of interest is selected from the group consisting of p-hydroxybenzaldehyde, p-hydroxybenzoic acid, bisoprolol, 4,4'-dihydroxydiphenylmethane, vanillin and polymers, especially liquid-crystalline polymer.

### BRIEF DESCRIPTION OF THE FIGURES

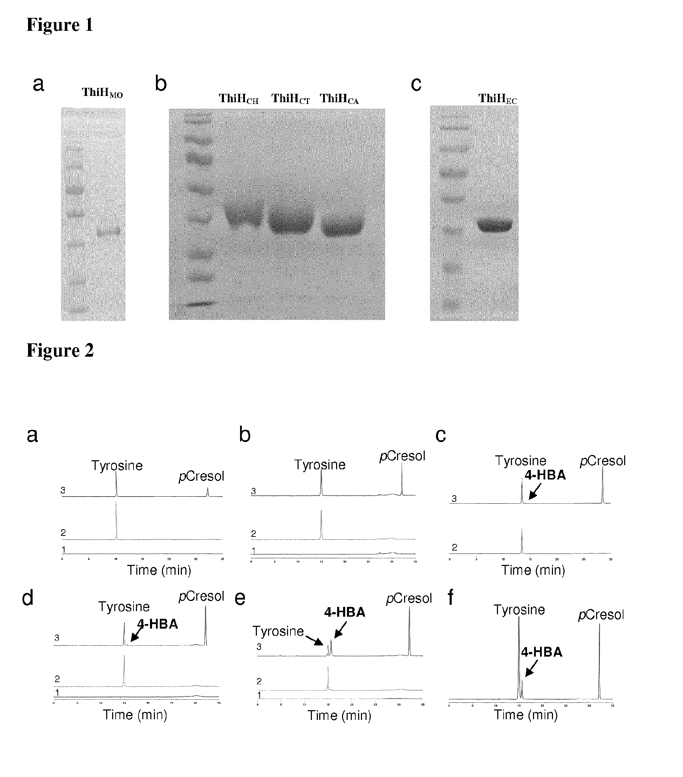

**Figure 1****:** Enzymes purification analyzed by SDS PAGE. ThiH from (a) *Moorella thermoacetica* (ThiH_{MO}), (b) *Carboxydothermus hydrogenoformans* (ThiH_{CH}), *Chlorobium tepidum* (ThiH_{CT}), *Clostridum acetobutylicum* (ThiH_{CA}) and (c) ThiH from *Escherichia coli* (ThiH_{EC}).
**Figure 2****:** ThiH activity with tyrosine as substrate under anaerobic and reducing conditions in the presence of *S*-adenosyl L-methionine (SAM) and dithionite from (a) *Clostridum acetobutylicum,* (b) *Chlorobium tepidum,* (c) *Escherichia coli,* (d) *Carboxydothermus hydrogenoformans,* (e) *Moorella thermoacetica* analyzed by HPLC compared to (f) reference compounds. Reactions were performed and analyzed (1) in the absence of tyrosine or with a full reaction medium at initial (2) and final (3) reaction times.

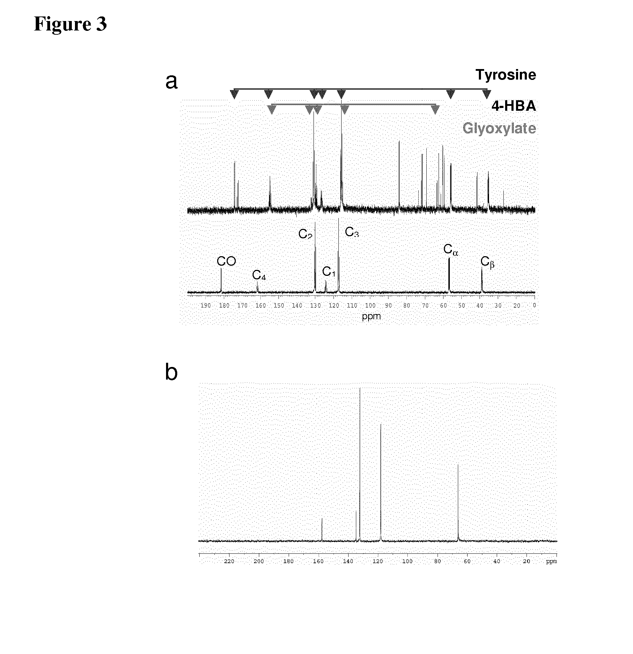

**Figure 3****:** NMR spectroscopy analysis of the enzymatic reaction with ThiH_{MO} (a) in the presence of ¹³C-labelled tyrosine showing the formation of 4-HBA. ¹³C-NMR analysis of the reaction with ThiH_{MO} (upper trace) and reference spectrum of ¹³C-tyrosine (lower trace). (b) Reference ¹³C-NMR spectrum of 4-hydroxy benzyl alcohol (4-HBA).

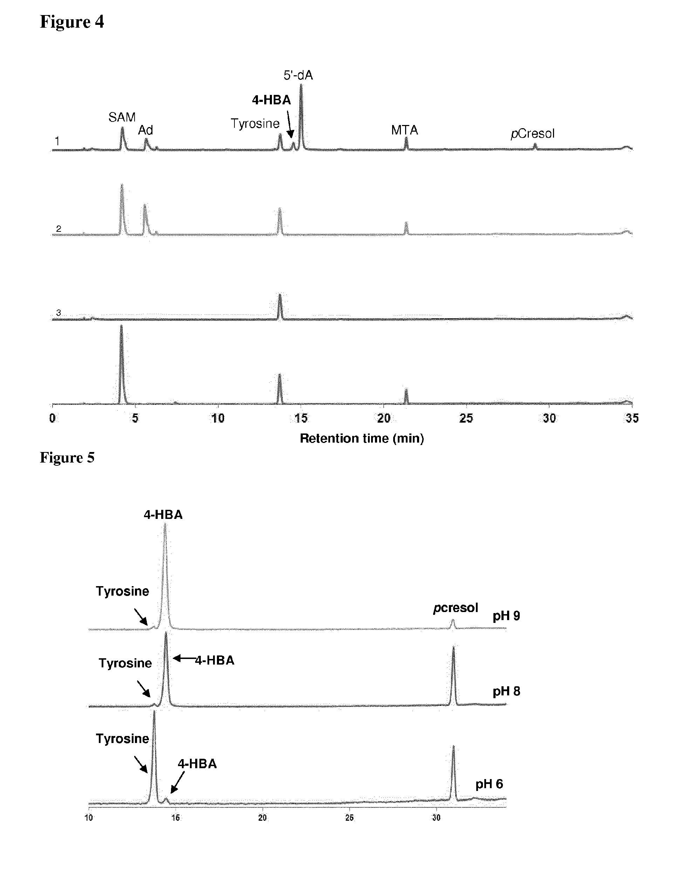

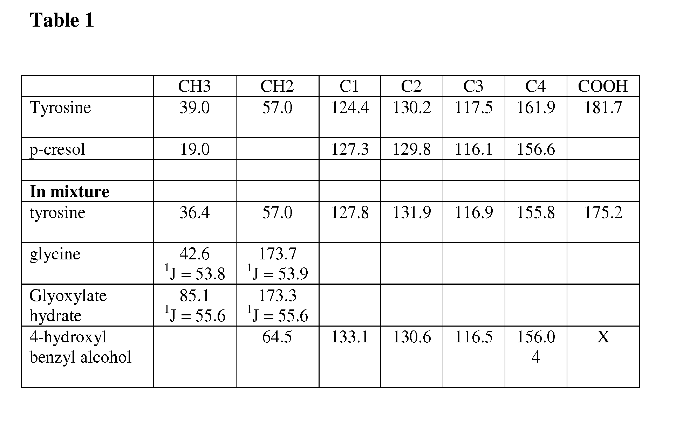

**Figure 4****:** C₁₈ HPLC analysis of the reaction of ThiH_{MO} under anaerobic conditions after 12h of incubations at 25°C (275nm) - The reaction was performed with (1) ThiH_{MO} (40µM), SAM (1mM), tyrosine (1 mM) and sodium dithionite as one-electron donor (2 mM) in Tris buffer pH 7.5 or in the absence of (2) sodium dithionite, (3) SAM or (4) ThiH_{MO}. SAM degradation products such as adenine (Ad) or methylthioadenosine (MTA) are formed independently of the enzymatic reaction.
**Figure 5****:** pH-dependent activity of ThiH_{MO} analyzed by HPLC and fluorescence. ThiH (40 µM) was incubated under anaerobic and reducing conditions in the presence of S-adenosyl L-methionine (1 mM), dithionite (2 mM) and tyrosine (1 mM).

**Table 1 -** ¹³C-NMR chemical shifts of tyrosine and p-cresol and measured ¹³C-NMR chemical shifts of ¹³C-labelled tyrosine, glycine, glyoxylate hydrate and 4-HBA in the mixture. CH₂ of tyrosine was set at 57.ppm.

### DETAILED DESCRIPTION OF THE INVENTION

The inventors surprisingly identified an enzyme, which specifically converts the amino acid tyrosine into p-cresol and 4-HBA (Figure 2-4 and Table 1). Although p-cresol is not of particular interest, its properties make the purification process of 4-HBA very straightforward.

Furthermore, the p-cresol/4-HBA ratio can be modified by the reaction conditions. Notably, a basic pH, preferably in the range of pH 7-10, improves the yield of 4-HBA and should be preferentially chosen. The reaction can be performed with standard enzyme buffers including non-exclusively phosphate, Tris and Borax buffers (Figure 5).

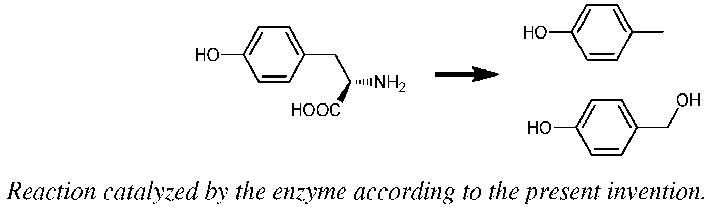

Furthermore, contrary to the standard chemical processes, this enzymatic synthesis does not lead to different HBAs or side products.

It is thus possible to produce 4-HBA in one step, without any organic solvent or toxic chemicals contrary to the currently available industrial processes. In summary: (a) ThiH_{MO} allows for a totally sustainable production of 4-HBA and (b) this enzyme produces 4-HBA safely, notably for medical and food applications.

### Definitions

*Coding sequence:* The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

*Control sequences:* The term "control sequences" means nucleic acid sequences necessary for expression of a polynucleotide encoding an enzyme of the present invention. Control sequences may be native (i.e., from the same gene) or heterologous (i.e., from a different gene and/or a different species) to the polynucleotide encoding the enzyme. Preferably, control sequences are heterologous. Well-known control sequences and currently used by the person skilled in the art will be preferred. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding the enzyme. The functional combination of control sequences and coding sequences can be referred as *expression cassette.*

*Expression:* The term "expression" includes any step involved in the production of a polypeptide including, but not limited to, transcription, post-transcriptional modification, translation, post-translational modification, and secretion.

*Expression vector:* The term "expression vector" means a linear or circular DNA molecule that comprises a polynucleotide encoding the enzyme of the invention and is operably linked to control sequences that provide for its expression. Then the expression vector comprises an expression cassette suitable for expressing the enzyme of the invention.

*Isolated:* The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., multiple copies of a gene encoding the substance; use of a stronger promoter than the promoter naturally associated with the gene encoding the substance).

*Recombinant:* Recombinant refers to a nucleic acid construct, a vector and a protein produced by genetic engineering.

*Heterologous:* in the context of a host cell, a vector or a nucleic acid construct, it designates a coding sequence for the enzyme introduced into the host cell, the vector or the nucleic acid construct by genetic engineering. In the context of a host cell, it can mean that the coding sequence for the enzyme originates from a source different from the cell in which it is introduced. Alternatively, it can also mean that the coding sequence for the enzyme comes from the same species as the cell in which it is introduced but it is considered heterologous due to its environment which is not natural, for example because it is under the control of a promoter which is not its natural promoter, or is introduced at a location which differs from its natural location.

*Nucleic acid construct:* The term "nucleic acid construct" means a nucleic acid molecule, either single- or double-stranded, which is modified to contain segments of nucleic acids in a manner that would not otherwise exist in nature or which is synthetic, which comprises one or more control sequences.

*Operably linked:* The term "operably linked" means a configuration in which a control sequence is placed at an appropriate position relative to a coding sequence, in such a way that the control sequence directs expression of the coding sequence.

*Sequence identity:* The sequence identity between two amino acid sequences is described by the parameter "sequence identity". For purposes of the present invention, the "percentage identity" between two amino acid sequences (A) and (B) is determined by comparing the two sequences aligned in an optimal manner, through a window of comparison. Said alignment of sequences can be carried out by well-known methods, for example, using the algorithm for global alignment of Needleman-Wunsch. Protein analysis software matches similar sequences using measures of similarity assigned to various substitutions, deletions and other modifications, including conservative amino acid substitutions. Once the total alignment is obtained, the percentage of identity can be obtained by dividing the full number of identical amino acid residues aligned by the full number of residues contained in the longest sequence between the sequence (A) and (B).

Sequence identity is typically determined using sequence analysis software. For comparing two amino acid sequences, one can use, for example, the tool "Emboss needle" for pairwise sequence alignment of proteins providing by EMBL-EBI and available on: www.ebi.ac.uk/Tools/services/web/toolform.ebi?tool=emboss_needle&context=protein, using default settings : (I) Matrix : BLOSUM62, (ii) Gap open : 10, (iii) gap extend : 0.5, (iv) output format : pair, (v) end gap penalty : false, (vi) end gap open : 10, (vii) end gap extend : 0.5.

*Variant:* The term "variant" means an enzyme capable of producing 4-HBA and p-cresol from L-tyrosine and comprising an alteration, i.e., a substitution, insertion, and/or deletion, at one or more (e.g., several) positions. In particular, the variant may have alterations at not more than 11, 10, 9, 8, 7, 6, 5, 4, 3, 2 or 1 amino acids, e.g., may have substitution, insertion, and/or deletion of 1, 2, 3, 4, 5, 6, 7, 8, 9 or 10 amino acids. A substitution means replacement of the amino acid occupying a position with a different amino acid; a deletion means removal of the amino acid occupying a position; and an insertion means adding an amino acid adjacent to and immediately following the amino acid occupying a position. The substitution can be a conservative substitution. Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill (1979, In, The Proteins, Academic Press, New York). Common substitutions are the followings Ala/Ser, Val/lle, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/lle, LeuA al, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like. Essential amino acids in a polypeptide can be identified according to procedures known in the art, such as site-directed mutagenesis or alanine-scanning mutagenesis (Cunningham and Wells, 1989, Science 244: 1081 -1085). In the latter technique, single alanine mutations are introduced at every residue in the molecule, and the resultant mutant molecules are tested for the capacity to produce 4-HBA from L-tyrosine to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for instance, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner ei a/., 1988, DNA 7: 127). Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

*Tyrosine analogs:* The tyrosine analogs refer to any analog of tyrosine capable of being converted by the enzyme of the invention. In particular, a tyrosine analog can be an analog of D-tyrosine, L-tyrosine or DL-tyrosine, preferably L-tyrosine. Preferably, the tyrosine analog has one or two substituents on the hydrobenzyl moiety or is an isomer of tyrosine. For instance, the tyrosine analog can be selected in the group consisting of the compounds O-Methyl-D-tyrosine (CAS No 39878-65-4), O-Methyl-L-tyrosine (CAS No 6230-11-1), O-Methyl-DL-tyrosine, O-Benzyl-D-tyrosine (CAS No 65733-15-5), O-Benzyl-L-tyrosine (CAS No 16652-64-5), O-Acetyl-L-tyrosine (CAS No 6636-22-2), O-2,6-Dichlorobenzyl-D-tyrosine, O-2,6-Dichlorobenzyl-L-tyrosine (CAS No 40298-69-9), O-tert-Butyl-D-tyrosine (CAS No 186698-58-8), O-tert-Butyl-L-tyrosine (CAS No 18822-59-8), L-meta-Tyrosine (CAS No 587-33-7), D-meta-Tyrosine (CAS No 32140-49-1), DL-meta-Tyrosine, DL-o-Tyrosine (CAS No 2370-61-8), L-2-Hydroxyphenylalanine (CAS No 7423-92-9), m-Iodo-L-tyrosine (CAS No 70-78-0), O-Phospho-L-tyrosine (CAS No 21820-51-9), 3,5-Diiodo-D-tyrosine (CAS No 16711-71-0), 3,5-Diiodo-L-tyrosine (CAS No 300-39-0), 3,5-Dinitro-D-tyrosine (CAS No 779321-23-2), 3,5-Dinitro-L-tyrosine (CAS No 17360-11-1), 3-Amino-L-tyrosine (CAS No 23279-22-3), 3-Chloro-D-tyrosine (CAS No 162599-96-4), 3-Chloro-L-tyrosine (CAS No 7423-93-0), 3-Fluoro-DL-tyrosine (CAS No 139-26-4), 3-Iodo-D-tyrosine (CAS No 25799-58-0), 3-Nitro-D-tyrosine (CAS No 32988-39-9), 3-Nitro-L-tyrosine (CAS No 621-44-3), D-3,5-Dibromotyrosine (CAS No 50299-42-8), L-3,5-Dibromotyrosine (CAS No 300-38-9), L-Homotyrosine (CAS No 141899-12-9), and D-Homotyrosine (CAS No 185617-14-5), preferably in the group consisting of the compounds O-Methyl-L-tyrosine (CAS No 6230-11-1), O-Benzyl-L-tyrosine (CAS No 16652-64-5), O-Acetyl-L-tyrosine (CAS No 6636-22-2), OO-2,6-Dichlorobenzyl-L-tyrosine (CAS No 40298-69-9), O-tert-Butyl-L-tyrosine (CAS No 18822-59-8), L-meta-Tyrosine (CAS No 587-33-7), L-2-Hydroxyphenylalanine (CAS No 7423-92-9), m-Iodo-L-tyrosine (CAS No 70-78-0), O-Phospho-L-tyrosine (CAS No 21820-51-9), 3,5-Diiodo-L-tyrosine (CAS No 300-39-0), 3,5-Dinitro-L-tyrosine (CAS No 17360-11-1), 3-Amino-L-tyrosine (CAS No 23279-22-3), 3-Chloro-L-tyrosine (CAS No 7423-93-0), 3-Nitro-L-tyrosine (CAS No 621-44-3), L-3,5-Dibromotyrosine (CAS No 300-38-9), and L-Homotyrosine (CAS No 141899-12-9),. These analogs are commercially available, for instance at Chem-Impex International Inc.

### Enzyme

It is provided an enzyme capable of producing 4-HBA and p-cresol in presence of L-tyrosine. Indeed, the inventors identified a tyrosine lyase ThiH from *Moorella thermoacetica* having the amino acid sequence of SEQ ID No 2. The enzyme is surprisingly capable of producing 4-HBA and p-cresol in presence of L-tyrosine and the co-factor S-adenosyl-L-methionine (SAM) following the reaction :

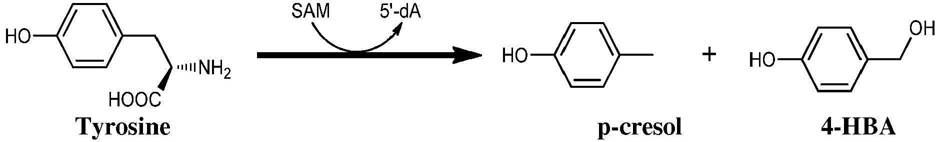

NMR analysis of the reaction demonstrated, starting from tyrosine, that the enzyme produces besides the expected molecules *i.e.* glyoxylate, p-cresol and glycine and a novel compound: 4-HBA, which has never been reported for such type of enzymes (Figure 3).

The p-cresol/4-HBA ratio is influenced by the reaction conditions; notably the pH affects strongly this ratio. To favor 4-HBA production, the pH should be between pH 7 and 10, as illustrated in Figure 5.

Therefore, it is provided an isolated or recombinant enzyme capable of producing 4-HBA and p-cresol in presence of L-tyrosine and comprising an amino acid sequence having at least 60 % identity with SEQ ID No 2. Preferably, the isolated or recombinant enzyme comprises or consists of an amino acid sequence having at least 80, 85, 90, 95, 97, 98, 99 % identity with SEQ ID No 2. In a very particular aspect, the isolated or recombinant enzyme comprises or consists of the amino acid sequence of SEQ ID No 2.

Because of their homologies with ThiH, the other radical SAM tyrosine lyases, CofH (26.9% similarity) involved in the biosynthesis of F₄₂₀ cofactor (Decamps et al. (2012) J Am Chem Soc 134, 18173-18176.) and HydG (44.2% similarity) involved in the H-cluster biosynthesis (Nicolet et al. (2009) FEBS Lett.;584(19):4197-202.), are also likely to be able to produce 4-HBA, either naturally or through enzyme engineering.

A method for testing the capacity of an enzyme to produce 4-HBA from L-tyrosine is for instance disclosed in details in the example section. More specifically, the enzyme is contacted with L-tyrosine in presence of the co-factor S-adenosyl-L-methionine (SAM) and the production of 4-HBA is detected. More particularly, the enzyme is capable of producing 4-HBA and p-cresol with a ratio ranging from between 1:30 to 30:1, preferably between 1:10 to 10:1, still more preferably between 2:3 and 3:2.

Based on the teaching of the present disclosure, the one skilled in the art can identify other enzymes from microorganisms having the 4-HBA producing activity from L-tyrosine. The polypeptide may be identified and obtained from other sources including microorganisms isolated from nature (e.g., soil, composts, water, etc.) or DNA samples obtained directly from natural materials (e.g., soil, composts, water, etc.) using the probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected, the polynucleotide can be isolated or cloned by utilizing techniques that are known to those of ordinary skill in the art (see, e.g., Sambrook et al., 1989). In addition, the person skilled in the art can prepare variants of the ThiH from *Moorella thermoacetica* having the amino acid sequence of SEQ ID No 2 by currently used methods. In particular, variants with advantageous properties such as an increased stability (e.g., thermostability), increased production of 4-HBA relative to p-cresol (e.g., improved ratio of 4-HBA/p-cresol).

It is also provided a hybrid polypeptide or fusion polypeptide in which the amino acid sequence of the enzyme as defined above is fused at the N-terminus or the C-terminus of a region of another polypeptide. Techniques for producing fusion polypeptides are known in the art, and include ligating the coding sequences encoding the enzyme and the addition region of another polypeptide so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

The addition region of the fusion polypeptide can be selected in order to enhance the stability of the enzyme according to the present disclosure, to promote the secretion (such as a N-terminal hydrophobic signal peptide) of the fusion protein from a cell (such as a bacterial cell or a yeast cell), or to assist in the purification of the fusion protein. More particularly, the additional region can be a tag useful for purification or immobilization of the enzyme. Such a tag is well-known by the person skilled in the art, for instance a His tag (His₆), a FLAG tag, a HA tag (epitope derived from the Influenza protein haemagglutinin), a maltose-binding protein (MPB), a MYC tag (epitope derived from the human proto-oncoprotein MYC), a STREP tag or a GST tag (small glutathione-S-transferase).

A fusion polypeptide can further comprise a cleavage site between the enzyme and the addition region. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

### Nucleic acid constructs

The present invention relates to a polynucleotide encoding an enzyme of the present invention. The nucleic acid can be DNA (cDNA or gDNA), RNA, or a mixture of the two. It can be in single stranded form or in duplex form or a mixture of the two. It can comprise modified nucleotides, comprising for example a modified bond, a modified purine or pyrimidine base, or a modified sugar. It can be prepared by any method known to one skilled in the art, including chemical synthesis, recombination, and mutagenesis. In particular, such a polynucleotide is disclosed in SEQ ID No 1.

The present invention also relates to nucleic acid constructs comprising a polynucleotide encoding an enzyme according to the present disclosure operably linked to one or more control sequences that direct the expression of the coding sequence in a suitable host cell under conditions compatible with the control sequences. A polynucleotide may be manipulated in a variety of ways to provide for expression of the enzyme. Manipulation of the polynucleotide prior to its insertion into a vector may be desirable or necessary depending on the expression vector. The techniques for modifying polynucleotides utilizing recombinant DNA methods are well known in the art.

The control sequence may include a promoter that is recognized by a host cell or an *in vitro* expression system for expression of a polynucleotide encoding an enzyme of the present invention. The promoter contains transcriptional control sequences that mediate the expression of the enzyme. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters in a bacterial host cell are the promoters obtained from the *Bacillus amyloliquefaciens* alpha-amylase gene (amyQ), *Bacillus licheniformis* alpha-amylase gene (amyL), *Bacillus licheniformis* penicillinase gene (penP), *Bacillus stearothermophilus* maltogenic amylase gene (amyM), *Bacillus subtilis* levansucrase gene (sacB), *Bacillus subtilis* xylA and xylB genes, *Bacillus thuringiensis* crylllA gene (Agaisse and Lereclus, 1994, Molecular Microbiology 13: 97-107), *E. coli* lac operon, *E. coli* trc promoter (Egon et al., 1988, Gene 69: 301 -315), *Streptomyces coelicolor* agarase gene (dagA), and prokaryotic beta-lactamase gene (Villa-Kamaroff et al., 1978, Proc. Natl. Acad. Sci. USA 75: 3727-3731), as well as the tac promoter (DeBoer et al., 1983, Proc. Natl. Acad. Sci. USA 80: 21 -25). Further promoters are described in "Useful proteins from recombinant bacteria" in Gilbert et al., 1980, Scientific American 242: 74-94; and in Sambrook et al., 1989. Examples of tandem promoters are disclosed in WO 99/43835.

Examples of suitable promoters in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (glaA), *Aspergillus oryzae* TAKA amylase, *Aspergillus oryzae* alkaline protease, *Aspergillus oryzae* triose phosphate isomerase, *Fusarium oxysporum* trypsin-like protease (WO96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene; and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae 3-*phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease (aprH), *Bacillus licheniformis* alpha-amylase (amyL), and *Escherichia coli* ribosomal RNA (rrnB).

Preferred terminators for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos et al., 1992, supra.

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis* crylllA gene (WO 94/25612) and a *Bacillus subtilis* SP82 gene (Hue et ai, 1995, Journal of Bacteriology 177: 3465-3471).

The control sequence may also be a leader, a non-translated region of an mRNA that is important for translation by the host cell. The leader is operably linked to the 5'-terminus of the polynucleotide encoding the enzyme. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide encoding the enzyme and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of the enzyme and directs the enzyme into the cell's secretory pathway. The 5'-end of the coding sequence of the polynucleotide may inherently contain a signal peptide coding sequence naturally linked in translation reading frame with the segment of the coding sequence that encodes the enzyme. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 1 1837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (nprT, nprS, nprM), and *Bacillus subtilis* prsA. Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos et al., 1992, *supra.*

It may also be desirable to add regulatory sequences that regulate expression of the polypeptide relative to the growth of the host cell. Examples of regulatory systems are those that cause expression of the gene to be turned on or off in response to a chemical or physical stimulus, including the presence of a regulatory compound. Regulatory systems in prokaryotic systems include the lac, tac, and trp operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked with the regulatory sequence.

### Expression vectors

The present invention also relates to recombinant expression vectors comprising a nucleic acid construct as disclosed above, or a polynucleotide encoding an enzyme of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the enzyme at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extra-chromosomal entity, the replication of which is independent of chromosomal replication, e.g., a plasmid, an extra-chromosomal element, a mini-chromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophy, and the like.

Examples of bacterial selectable markers are *Bacillus licheniformis* or *Bacillus subtilis* genes or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, amdS (acetamidase), argB (ornithine carbamoyltransferase), bar (phosphinothricin acetyltransferase), hph (hygromycin phosphotransferase), niaD (nitrate reductase), pyrG (orotidine-5'-phosphate decarboxylase), sC (sulfate adenyltransferase), and trpC (anthranilate synthase), as well as equivalents thereof. Preferred for use in an *Aspergillus* cell are *Aspergillus nidulans* or *Aspergillus oryzae* amdS and pyrG genes and a *Streptomyces hygroscopicus* gene.

The vector preferably contains an element(s) that permits integration of the vector into the host cell's genome or autonomous replication of the vector in the cell independent of the genome.

When integration into the host cell genome occurs, integration of the sequences into the genome may rely on homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate in vivo. Examples of bacterial origins of replication are the origins of replication of plasmids pBR322, pUC19, pACYC177, and pACYC184 permitting replication in *E. coli,* and pUB1 10, pE194, pTA1060, and pAMβ1 permitting replication in Bacillus. Examples of origins of replication for use in a yeast host cell are the 2 micron origin of replication, ARS1, ARS4, the combination of ARS1 and CEN3, and the combination of ARS4 and CEN6. Examples of origins of replication useful in a filamentous fungal cell are AMA1 and ANSI (Gems et al., 1991, Gene 98: 61 -67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, e.g., Sambrook et al., 1989, supra).

### Host cells

The present invention also relates to recombinant host cells, comprising a polynucleotide encoding the enzyme according to the present disclosure operably linked to one or more control sequences that direct the production of the enzyme of the present invention. A construct or vector comprising a polynucleotide encoding the enzyme of according to the present disclosure is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, e.g., a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.* The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells. The bacterial host cell may also be any *Streptococcus* cell including, but not limited to, *Streptococcus equisimilis, Streptococcus pyogenes, Streptococcus uberis, Streptococcus equi* and *Streptococcus zooepidemicus* cells. The bacterial host cell may further be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, e.g., Chang and Cohen, 1979, Mol. Gen. Genet. 168: 111-115), competent cell transformation (see, e.g., Young and Spizizen, 1961, J. Bacteriol. 81: 823-829, or Dubnau and Davidoff-Abelson, 1971, J. Mol. Biol. 56: 209-221), electroporation (see, e.g., Shigekawa and Dower, 1988, Biotechniques 6: 742-751), or conjugation (see, e.g., Koehler and Thorne, 1987, J. Bacteriol. 169: 5271 -5278). The introduction of DNA into an *E. coli* cell may be effected by protoplast transformation (see, e.g., Hanahan, 1983, J. Mol. Biol. 166: 557-580) or electroporation (see, e.g., Dower et al, 1988, Nucleic Acids Res. 16: 6127-6145). The introduction of DNA into a *Streptomyces* cell may be effected by protoplast transformation, electroporation (see, e.g., Gong et al., 2004, Folia Microbiol. (Praha) 49: 399-405), conjugation (see, e.g., Mazodier ei a/., 1989, J. Bacteriol. 171: 3583-3585), or transduction (see, e.g., Burke et al., 2001, Proc. Natl. Acad. Sci. USA 98: 6289-6294). The introduction of DNA into a *Pseudomonas* cell may be effected by electroporation (see, e.g., Choi et al., 2006, J. Microbiol. Methods 64: 391 -397) or conjugation (see, e.g., Pinedo and Smets, 2005, Appl. Environ. Microbiol. 71: 51 -57). The introduction of DNA into a *Streptococcus* cell may be effected by natural competence (see, e.g., Perry and Kuramitsu, 1981, Infect. Immun. 32: 1295-1297), protoplast transformation (see, e.g., Catt and Jollick, 1991, Microbios 68: 189-207), electroporation (see, e.g., Buckley et al., 1999, Appl. Environ. Microbiol. 65: 3800-3804), or conjugation (see, e.g., Clewell, 1981, Microbiol. Rev. 45: 409-436). However, any method known in the art for introducing DNA into a host cell can be used.

The host cell may also be a eukaryote, such as a mammalian, insect, plant, or fungal cell. The host cell may be a fungal cell. "Fungi" as used herein includes the phyla *Ascomycota, Basidiomycota, Chytridiomycota,* and *Zygomycota* as well as the *Oomycota* and all mitosporic fungi (as defined by Hawksworth et al., In, Ainsworth and Bisby's Dictionary of The Fungi, 8th edition, 1995, CAB International, University Press, Cambridge, UK). The fungal host cell may be a yeast cell. "Yeast" as used herein includes ascosporogenous yeast (Endomycetales), basidiosporogenous yeast, and yeast belonging to the *Fungi imperfecti* (Blastomycetes). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980). The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell. The fungal host cell may be a filamentous fungal cell. "Filamentous fungi" include all filamentous forms of the subdivision *Eumycota* and *Oomycota* (as defined by Hawksworth et al., 1995, supra). The filamentous fungi are generally characterized by a mycelial wall composed of chitin, cellulose, glucan, chitosan, mannan, and other complex polysaccharides. The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell. For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

The cell can also be a mammalian cell, for example COS, CHO (US 4,889,803; US 5,047,335). In a particular embodiment, the cell is non-human and non-embryonic. In addition, the enzyme of the invention could be produce by a non-human transgenic animal, for instance in the milk produces by the animal.

The cell can be a plant cell. Then, the enzyme of the invention could be produce by a transgenic plant.

A particular host cell of interest in the present disclosure is a host cell overproducing tyrosine or analog thereof, in particular L-tyrosine. In particular, the host cell can be a cell overproducing tyrosine, more preferably a genetically engineered host cell. Cells overproducing tyrosine are known. Several different microorganisms have been modified for L-Tyr production. *Corynebacterium glutamicum, Arthrobacter globiformis,* and *Brevibacterium lactofermentum* L-Tyr-overproducing strains were developed by classical mutagenesis methods (Ito et al., Agric Biol Chem. 1990 Mar; 54(3):699-705; Hagino, H., and K. Nakayama. 1973. Agric. Biol. Chem. 39:2013-2023; Roy, et al. 1997. J. Sci. Ind. Res. 56:727-733). Metabolic engineering and protein-directed evolution strategies have been used to construct *E. coli* L-Tyr-producing strains (US 2005/0277179; Lütke-Eversloh T, Stephanopoulos G. Appl Environ Microbiol. 2005 Nov; 71(11):7224-8; Lütke-Eversloh T, Stephanopoulos G. Appl Microbiol Biotechnol. 2007 May; 75(1):103-10; Patnaik Ret al. Biotechnol Bioeng. 2008 Mar 1; 99(4):741-52; Chavez-Bejar et al, Appl Environ Microbiol. 2008 May; 74(10): 3284-3290). Therefore, a host cell overproducing tyrosine and expressing (or being able to express under suitable conditions) the enzyme according to the present disclosure is of particular interest.

### Method of enzyme production

The present invention also relates to (a) methods of producing the enzyme of the present invention wherein a nucleic acid construct encoding the enzyme according to the present disclosure is expressed; and (b) recovering the enzyme.

In a first aspect, the present invention also relates to *in vitro* methods of producing the enzyme of the present invention wherein a nucleic acid construct as disclosed above is contacted with an *in vitro* expression system; and recovering the enzyme. The *in vitro* expression systems are well known to the person skilled in the art and are commercially available.

In a second aspect, the present invention also relates to methods of producing the enzyme of the present invention, comprising (a) culturing a cell, which in its wild-type form produces the enzyme according to the present disclosure, under conditions conducive for production of the enzyme; and (b) recovering the enzyme. In a preferred aspect, the cell is a *Moorella thermoacetica* cell. *Moorella thermoacetica* was previously known as *Clostridium thermoaceticum.*

In a third aspect, the present invention also relates to methods of producing the enzyme according to the present disclosure, comprising (a) cultivating a recombinant host cell as described above under conditions conducive for production of the enzyme; and (b) recovering the enzyme.

The host cells are cultivated in a nutrient medium suitable for production of polypeptides using methods known in the art. For example, the cell may be cultivated by shake flask cultivation, or small-scale or large-scale fermentation (including continuous, batch, fed- batch, or solid state fermentations) in laboratory or industrial fermentors performed in a suitable medium and under conditions allowing the enzyme to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the enzyme is secreted into the nutrient medium, the enzyme can be recovered directly from the medium. If the enzyme is not secreted, it can be recovered from cell lysates. The enzyme may be detected using methods known in the art that are specific for the enzyme. These detection methods include, but are not limited to, use of specific antibodies, detection of tag, formation of an enzyme product, or disappearance of an enzyme substrate. For example, an enzyme assay may be used to determine the activity of the enzyme.

The enzyme may be recovered using methods known in the art. For example, the enzyme may be recovered from the nutrient medium by conventional procedures including, but not limited to, collection, centrifugation, filtration, extraction, spray-drying, evaporation, or precipitation.

The enzyme may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, Janson and Ryden, editors, VCH Publishers, New York, 1989) to obtain substantially pure polypeptides. In an alternative aspect, the enzyme is not recovered, but rather a host cell of the present invention expressing the enzyme is used as a source of the enzyme.

In addition, it is also provided the use of the enzyme according to the present disclosure for preparing the enzyme immobilized on a solid support; and a method for preparing an enzyme as disclosed above immobilized on a solid support comprising producing the enzyme as detailed above and immobilizing the enzyme on a solid support.

The present invention also relates to a solid support, the enzyme according to the present invention being immobilized on the solid support. Immobilization means are well-know to the person skilled in the art ('Enzyme Technology' by Martin Chaplin and Christopher Bucke (Cambridge University Press, 1990); Lim et al. 2009, Process Biochemistry 44, 822-828 ; WO2011/040708; Alloue et al, Biotechnol Agron Soc Environ 2008, 12, 57-68; the disclosure thereof being incorporated herein by reference.. The enzyme according to the present disclosure can be immobilized on the solid support by any convenient mean, in particular adsorption adsorption, covalent binding, entrapment or membrane confinement. A wide variety of insoluble materials may be used to immobilize the enzyme. These are usually inert polymeric or inorganic matrices. For example, the enzyme can be immobilized on a polyurethane matrix (Gordon et al., 1999, Chemical-Biological Interactions 14:463-470) on activated sepharose, alginate, amberlite resin, Sephadex resin or Duolite resin. Other solid supports useful for the invention include resins with an acrylic type structure, polystyrene resins, macroreticular resins and resins with basic functional groups, such as Sepabeads EC-EP and Relizime (Resindion Srl, Mitsubishi Chemical Corporation) and Eupergit C (Röhm GmbH & Co. KG). In any case, the enzyme is brought in contact with the resin and is eitherimmobilized through the high reactivity of the functional groups or activation of the resin with a bifunctional agent, such as glutaraldehyde, so as to bind the enzyme to the matrix, or is absorbed on the resin and then stabilized by cross-linking with a bifunctional agent (glutaraldehyde). The solid support can be for instance membranous, particulate or fibrous. More particularly, the solid support is preferably a bead, e.g., micro- or nanobeads. Then, the enzyme is immobilized on a solid support in order to prepare a reactor, which can be for instance an enzyme reactor, a membrane reactor, a continuous flow reactor such as a stirred tank reactor, a continuously operated packed bed reactor, or a continuously operated fluidized bed reactor, or a packed bed reactor.

### Compositions and kits

The produced enzyme can be formulated in a composition. The composition comprises components suitable for enzyme preservation. The enzyme can be free or immobilized on a solid support, preferably beads. The composition can be liquid or dry. It comprises the enzyme according to the disclosure in a purified or enriched form. Liquid compositions preferably contain the enzyme in a purified or enriched form. However, auxiliaries such as a stabilizer like glycerol (also called glycerine), sorbitol or monopropylene glycol, additives like salts, sugar, preservatives, agents for to adjust the pH value (buffer), a redox agent such as DTT (dithiothreitol), or a sequester such as EDTA (ethylenediaminetetraacetic acid) can be added. In particular, the liquid composition can comprise at least 10, 20, 30, 40 or 50 % (w/v) of glycerol sorbitol or monopropylene glycol, preferably between 20 and 50 % (w/v). Preferably, the composition comprises glycerol. Optionally, the composition may further include the co-factor SAM. Typical liquid compositions are aqueous or oleaginous suspensions.

Therefore the present invention relates to a composition, especially an enzymatic composition, comprising the enzyme according to the present disclosure and appropriate auxiliaries, in particular those disclosed above. Preferably, the composition comprises, as enzymes or proteins component, at least 75, 80, 85, 90, 95 % enzyme.

It is also provided a kit for producing 4-HBA comprising an enzyme, a composition, a support solid with the immobilized enzyme or a host cell capable of expressing the enzyme as described above. The kit may further comprise other reagents such as SAM, buffer and a reducing agent: a source of one-electron donor such as sodium dithionite, methyl viologen or an enzymatic systems such as flavodoxin/ flavodoxin reductase/ NADPH, and addition of iron and sulfur if necessary.

### Methods and Uses

The present invention relates to the use of
- the enzyme according to the present disclosure; or
- the solid support with the immobilized enzyme; or
- the host cell capable of expressing the enzyme; or
- a kit as disclosed above;
for producing 4-HBA or an analog thereof, or a compound of interest prepared from 4-HBA or the analog thereof, preferably for producing 4-HBA or a compound of interest prepared from 4-HBA.

It also relates to a method for producing 4-HBA or an analog thereof comprising contacting tyrosine or an analog thereof with an enzyme comprising an amino sequence having at least 80 % identity with SEQ ID No 2 and being capable of producing 4-HBA and p-cresol from L-tyrosine, and optionally recovering 4-HBA or the analog thereof.

Preferably, the tyrosine or an analog thereof has the following formula: 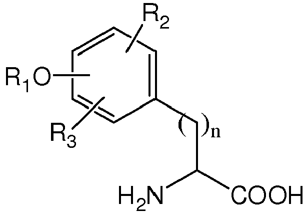 wherein n is 0, 1 or 2, preferably 1
R1 is selected from the group consisting of a hydrogen, a C1-C4 alkyl, an aryl, an C1-C3alkylaryl, a C1-C4 acyl, and a phosphate, preferably from the group consisting of methyl, ethyl, t-butyl, phenyl, benzyl and acetyl;
and R2 and R3, independently from each other, can be selected from the group consisting of a hydrogen, a halogen (preferably chloro, iodo, bromo or fluroro), a C1-C4 alkyloxy (preferably methoxy or ethoxy), nitro, cyano, amino, amide, and trifluoromethyl.

The tyrosine or the analog can be L or D, preferably L.

Preferably, the 4-HBA and an analog thereof has the following formula 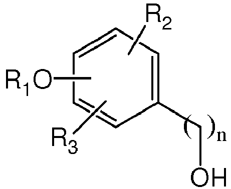 wherein n, R1, R2 and R3 have the same definition as above.

OR1 can be in position ortho, meta or para. Preferably, OR1 is in para.

Preferably, R2 and/or R3 are in position meta.

More particularly, the tyrosine or an analog thereof has the following formula: 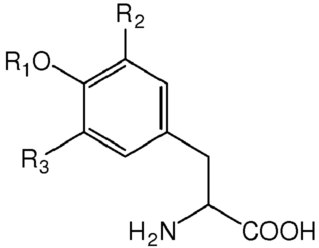 and the 4-HBA or an analog thereof has the following formula 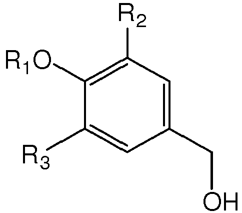 wherein R1, R2 and R3 have the same definition than above.

In a preferred and particular embodiment, R1, R2 and R3 are hydrogen atoms.

Preferably, the tyrosine or an analog thereof is contacted with the enzyme in the presence of the SAM cofactor. The reaction, for *in vitro* production, is preferably performed under anaerobic and reducing conditions between pH 6 and 10. A source of one-electron donor will be preferably present, for instance, but not limited to, chemical agents such as dithionite, methyl viologen or enzymatic systems such as flavodoxin/ flavodoxin reductase/ NADPH. The reaction is preferentially performed between 20°C and 40°C but higher or lower temperatures might be used. The standard reaction is performed with ThiH_{MO} (40µM), SAM (1mM), tyrosine (1 mM), dithiothreitol (6 mM) and sodium dithionite (2 mM) in Tris buffer pH 8 under anaerobic conditions.

The method may comprise a further step of purification of the 4-HBA or the analog thereof. More specifically, 4-HBA and the by-product p-cresol can be easily separated in order to recover/purify 4-HBa. Indeed, the two compounds have very different hydrophobicity. They can be separated by any convenient method well known to the skilled person, for instance hydrophobic interaction chromatography (HIC), solid phase extraction (SPE), or distillation.

It is also provided an alternative method for producing 4-HBA comprising culturing a host cell as defined above, preferably a host cell overproducing tyrosine, and optionally recovering 4-HBA.

The present invention further relates to a method for preparing a compound of interest that comprises the production of 4-HBA, or an analog thereof, by a method according to the present invention and using the 4-HBA or the analog thereof for preparing the compound of interest. Such compound of interest is any compound that can be prepared from 4-HBA or an analog thereof, but preferably from 4-HBA. For instance, the compound of interest could be p-hydroxybenzaldehyde and p-hydroxybenzoic acid by 4-HBA oxidation (Garade et al. (2001) Catalysis Communications 10 (2009) 485-489), bisoprolol (WO2007/069266), 4,4'-dihydroxydiphenylmethane or polymers, especially liquid-crystalline polymer (e.g., US2012/190813) by condensation, or vanillin. In addition, as 4-HBA is of therapeutically interest, a formulation of 4-HBA can be also prepared such as a p-hydroxybenzyl alcohol-containing biodegradable polyoxalate nanoparticulate antioxidant (Kim et al, Biomaterials, 2011, 32(11):3021-9).

### EXAMPLES

**ThiH cloning.** Genes coding for tyrosine lyases (ThiH) variants from different organisms i.e. *Moorella thermoacetica* (MO), *Carboxythermus hydrogenoformans* (CH), *Escherichia coli, Clostridium acetobutylicum* (CA) *and Chlorobium tepidum,* were either cloned or synthesized and inserted into a suitable expression vector. Sequence-optimized synthetic genes of ThiH_{MO}, ThiH_{CH} and ThiH_{CA} were obtained from GenScript™ and were inserted into a pET-15b (Novagen^{®}) vector between NdeI and BamHI restriction sites. The *Thih_{CT}* gene was amplified by a standard PCR protocol using 5'-GGTAATCCATATGATTGCGCTGCCCGCATGGCTGACC-3' (SEQ ID No 11) and 5'-GGGAATTCTTATCACGTGCACTCCTCTGCGGGCAGG-3' (SEQ ID No 12) oligonucleotides as primers and Phusion™ as polymerase. The amplified fragment was subsequently inserted into a pET-28a vector (Novagen^{®}) between NdeI and EcoRI restriction sites. *Thih_{EC},* was cloned using standard PCR protocols and inserted into a pASK-=17plus vector. The integrity of the cloned sequences was determined by sequencing the entire genes.

**ThiH expression and purification.** *E. coli* BL21(DE3) cells were transformed with pET15b-ThiH (or pET28a-TiH or pASK17plus-ThiH) and grown aerobically overnight at 37°C in LB medium supplemented with ampicillin (100 µg.mL-1). An overnight culture was then used to inoculate fresh LB medium supplemented with the same antibiotic and bacterial growth proceeded at 37°C until the OD₆₀₀ reached 0.6. The cells were induced by adding 200µM IPTG and collected after overnight growth at 20°C. After re-suspension in Tris-buffer (50 mM Tris, 300 mM KCl, 10mM MgCl₂, 500 mM NaCl, pH 7.5), the cells were disrupted by sonication and centrifuged at 220,000 x g at 4°C for 90 minutes. The solution was then loaded onto a Ni-NTA Sepharose column previously equilibrated with Tris-buffer. The column was washed extensively with the same buffer. Three elution steps were performed at 25mM, 75mM and 500 mM imidazole in Tris-buffer. The over-expressed protein was eluted in the 500 mM imidazole fraction. Fractions containing ThiH were immediately desalted on a PD10 column (GE Healthcare) with Tris-buffer as eluent, concentrated in Amicon Ultra-4 (Millipore) with a molecular cut-off of 10 kDa and frozen in liquid nitrogen.

For ThiH expressed with the pASK17plus plasmid, a similar protocol was used but cells were induced by 200µg.L⁻¹ anhydrotetracycline and the enzyme was purified using a Strep-Tactin resin equilibrated with Tris-buffer (50 mM Tris, 300 mM KCl, 10mM MgCl₂, 500 mM NaCl, pH 7.5). Elution was performed using the same buffer containing 3 mM dethiobiotin.

Protein concentrations were determined by the Bradford protein assay, using BSA as a standard. The collected fractions were analyzed by 12% polyacrylamide gel electrophoresis under denaturing conditions (SDS-PAGE).

**Reconstitution of Fe-S clusters.** Reconstitution of Fe-S clusters was carried out anaerobically in a glove box (Bactron IV). Purified ThiH (170 µM monomer) was treated with 6 mM DTT and then incubated at 12°C overnight with a 5-fold molar excess of both Na₂S (Fluka) and (NH4)₂Fe(SO₄)₂ (Aldrich). The protein was desalted using a Sephadex G25 column (Amersham) and the colored fractions were concentrated with an Amicon Ultra-4 (Millipore). Protein concentrations were determined by the Bradford protein assay, using BSA as a standard. Iron concentrations were determined colorimetrically using bathophenanthroline under reducing conditions (Fish, W. W. (1988) Methods Enzymol 158, 357-64).

**ThiH enzymatic assay.** The enzymatic assay was performed in an anaerobic glove box (Bactron IV) at 25°C. Samples contained 6 mM dithiothreitol, 3 mM sodium dithionite, 20 µM of reconstituted ThiH, along with 1 mM tyrosine and 1 mM SAM in Tris-buffer, pH 7.5. Control samples were prepared without enzyme to check tyrosine and SAM stability over time. Enzymatic assays were also performed using uniformly ¹³C-labeled tyrosine as substrate in the same conditions.

Reaction products were analyzed by HPLC using a C₁₈ column (LicroSphere, 5-µm, 4.6 x 150-mm) eluted at 1 mL/min with the following gradient: after a 1 ml step of Milli-Q H2O/ 0.1% trifluoroacetic acid, a three-step gradient from 0 to 9.6% in 17min, from 9.6% to 35.2% in 7 min and finally from 35.2 to 42.4% acetonitrile with 0.1% TFA in 10 min was used to elute the samples. Detection was carried out at 257 nm and 275 nm with a photodiode array detector. SAM, 5'-deoxyadenosine, tyrosine, p-cresol and dihydroxybenzyl alcohol were injected as standards.

### NMR analysis.

¹³C-NMR chemical shifts of ¹³C-labelled tyrosine and its derivatives, p-cresol, glycine, glyoxylate hydrate and 4-HBA was determined using a Bruker AVANCE III 600 MHz spectrometer equipped with a 5 mm 1H/13C/15N/31P QCI Z-Gradient Cryoprobe. The ¹³C NMR spectra with proton decoupling were recorded with 64K data points using a spectral width of 36 000 Hz in the mixture. An exponential weighting function was applied prior to Fourier transformation. No internal reference was added. The CH₂ of tyrosine was set at 57 ppm in the reaction medium as in the pure sample.

**Detection of 4-HBA as a novel by-product in ThiH.** UV-visible spectra of the five purified and *in vitro* Fe-S cluster reconstituted proteins exhibited typical Fe-to-S charge transfer bands at ~320 and ~420 nm consistent with the presence of one Fe₄S₄ center per polypeptide, as expected. These variants could be expressed and purified without ThiG in good yields contrary to the case of the *E. coli* enzyme. These five enzymes were assayed under identical conditions for tyrosine lyase activity. They were shown to catalyze efficient tyrosine cleavage and production of p-cresol in agreement with the current knowledge on ThiH enzymes. Unexpectedly, reacted mixtures from ThiH_{CH} and ThiH_{MO} exhibited an additional compound (compound 1) in the HPLC elution profile (rt ~15.5min), whose UV-visible and fluorescence spectra are consistent with a novel tyrosine derivative (Figure 2). Using ThiH_{MO}, which produces the highest amount of compound 1, the inventors observed that its formation strictly depends on the presence of SAM, tyrosine and a reducing agent. Compounds 1 is thus produced by the radical-based activity of ThiH and not by an hypothetic secondary activity of the enzyme. Using ¹³C-labeled tyrosine as substrate and ThiH_{MO}, the inventors were able to perform ¹³C-NMR experiments on the whole reaction mixture. The ¹³C-NMR spectrum analysis indicates the presence of four major compounds: tyrosine, glyoxylate, and, unexpectedly, glycine and 4-HBA (see figure 3). The latter exhibits modified chemical shifts compared to tyrosine, notably at C4 (157 vs. 155 pm), C1 (133 vs 129 ppm) and a major shift on the Cβ (64 vs. 37 ppm) in full agreement with experimentally measured values for 4-hydroxy benzyl alcohol, used as a standard. In addition, commercially available 4-HBA displays a retention time on HPLC, as well as UV-visible and fluorescence properties identical to compound 1. This univocally demonstrates that compound 1 corresponds to 4-HBA produced by ThiH. Furthermore, 4-HBA synthesis proved to be independent of the reducing system used since the *E*. *coli* physiological reduction system, flavodoxin/flavodoxin reducatase/NADPH also allowed for efficient production of 4-HBA. In full agreement with previous reports on ThiH from *E*. *coli* (ThiH_{EC}) the observed glyoxylate, results from the spontaneous hydrolysis of dehydroglycine, the precursor of the thiamine thiazole moiety. Its measured chemical shifts exactly matched those found for glyoxylate in the case of ThiH_{EC}.

In the case of ThiH_{MO}, addition of 5'-dA, p-cresol or 4-HBA leads to a significant inhibition of the reaction. Also, addition of 4-HBA or p-cresol did not lead to any changes in their relative concentrations, excluding the inter-conversion of these molecules by the enzyme. On the other hand, assaying the enzyme under different pH conditions changes the 4-HBA/p-cresol ratio from 0.3 at pH 6 to 6 at pH 9 (Figure 5). Either the protonation state of the enzyme influences the production of 4-HBA versus p-cresol, or the pH modifies the relative stability of the reaction intermediates that lead either to 4-HBA or p-cresol. In addition, as the redox potential of dithionite decreases with increasing pH, this change may also influence the enzymatic production of 4-HBA vs. p-cresol.

## Claims

1. A method for producing 4-hydroxyl benzyl alcohol (4-HBA) or an analog thereof comprising contacting tyrosine or an analog thereof with an enzyme comprising an amino sequence having at least 80 % of identity with SEQ ID No 2 and being capable of producing 4-HBA and p-cresol from L-tyrosine, and optionally recovering 4-HBA or the analog thereof.

2. The method of claim 1, wherein the enzyme comprises an amino sequence having at least 90, 95, 97.5 or 99 % identity with SEQ ID No 2.

3. The method of claim 1 or 2, wherein the enzyme comprises the amino acid sequence of SEQ ID No 2.

4. The method of any one of claims 1-3, wherein the enzyme is contacted with tyrosine, preferably L-tyrosine.

5. The method of any one of claims 1-4, wherein the enzyme is contacted with tyrosine or an analog thereof in presence of the co-factor S-adenosyl-L-methionine (SAM).

6. The method of any one of claims 1-5, wherein the method further comprises a purification step of 4-HBA or the analog thereof.

7. A recombinant nucleic acid construct or vector comprising an nucleic acid sequence encoding an enzyme comprising an amino sequence having at least 80 % identity with SEQ ID No 2 and being capable of producing 4-HBA and p-cresol from L-tyrosine.

8. A recombinant host cell comprising a recombinant nucleic acid construct or vector of claim 7 or a nucleic acid sequence encoding an enzyme comprising an amino sequence having at least 80 % identity with SEQ ID No 2 and being capable of producing 4-HBA and p-cresol from L-tyrosine.

9. A method for producing an enzyme capable of producing 4-HBA and p-cresol from L-tyrosine, comprising either cultivating the host cell of claim 8 under conditions conducive for production of the enzyme or expressing *in vitro* the enzyme with the nucleic acid construct or vector of claim 7, and recovering and/or purifying the enzyme.

10. A solid support on which is immobilized an enzyme comprising an enzyme comprising or consisting of an amino sequence having at least 80 % identity with SEQ ID No 2 and being capable of producing 4-HBA and p-cresol from L-tyrosine.

11. A kit for producing 4-HBA comprising a recombinant enzyme comprising or consisting of an amino sequence having at least 80 % identity with SEQ ID No 2 and being capable of producing 4-HBA and p-cresol from L-tyrosine, a solid support of claim 10 or a host cell of claim 8.

12. A composition comprising an enzyme comprising or consisting of an amino sequence having at least 80 % identity with SEQ ID No 2 and being capable of producing 4-HBA and p-cresol from L-tyrosine, and optionally further comprising S-adenosyl L-methionine, iron, sulfur, a reducing agent and/or another source of electrons.

13. Use for producing 4-HBA of an enzyme having at least 80 % identity with SEQ ID No 2 and being capable of producing 4-HBA and p-cresol from L-tyrosine, a host cell of claim 8, a solid support of claim 10, a kit of claim 11.

14. A method for producing a compound of interest, comprising producing 4-HBA or an analog thereof by the method according to any one of claims 1-6 and using the 4-HBA or the analog thereof for producing the compound of interest.

15. The method of claim 14, wherein the compound of interest is selected from the group consisting of p-hydroxybenzaldehyde, p-hydroxybenzoic acid, bisoprolol, 4,4'-dihydroxydiphenylmethane, vanillin and polymers, especially liquid-crystalline polymer.
